(19) Europäisches Patentamt / European Patent Office / Office européen des brevets

(11) **EP 4 487 902 B1**

(12) **EUROPEAN PATENT SPECIFICATION**

(45) Date of publication and mention
of the grant of the patent:
**02.07.2025  Bulletin 2025/27**

(21) Application number: **24176398.6**

(22) Date of filing: **16.05.2024**

(51) International Patent Classification (IPC):
**A61N 1/368** (2006.01)      **A61N 1/375** (2006.01)
A61B 5/00 (2006.01)      A61N 1/365 (2006.01)
A61N 1/372 (2006.01)

(52) Cooperative Patent Classification (CPC):
**A61N 1/3756; A61N 1/368;** A61B 5/686;
A61N 1/36507; A61N 1/37288

(54) **SYSTEMS FOR REDUCING COMMUNICATION BURDEN BETWEEN LEADLESS PACEMAKERS**

SYSTEME  ZUR REDUZIERUNG DER KOMMUNIKATIONSLAST ZWISCHEN LEITUNGSLOSEN HERZSCHRITTMACHERN

SYSTÈMES POUR RÉDUIRE LA CHARGE DE COMMUNICATION ENTRE DES STIMULATEURS CARDIAQUES SANS FIL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **05.07.2023   US 202363511998 P**
**15.05.2024   US 202418665315**

(43) Date of publication of application:
**08.01.2025   Bulletin 2025/02**

(73) Proprietor: **Pacesetter, Inc.**
**Sylmar, CA 91342 (US)**

(72) Inventor: **Fishler, Matthew G**
**Scotts Valley, CA (US)**

(74) Representative: **Barker Brettell Sweden AB**
**Kungsbroplan 3**
**112 27 Stockholm (SE)**

(56) References cited:
**US-A1- 2016 121 128      US-A1- 2016 310 733**
**US-A1- 2021 205 628      US-A1- 2021 370 078**

**Description**

FIELD OF TECHNOLOGY

Embodiments described herein generally relate to dual chamber leadless pacemaker systems, leadless pacemakers thereof, and methods for use by dual chamber leadless pacemaker systems and leadless pacemakers thereof.

BACKGROUND

A dual chamber leadless pacemaker (LP) system can include an atrial leadless pacemaker (aLP) and a ventricular leadless pacemaker (vLP) that utilize implant-to-implant (i2i) communication to coordinate their dual-chamber functionality. More specifically, such a dual chamber LP system may utilize an i2i communication protocol that requires the aLP to transmit an i2i event message to the vLP whenever the aLP senses an intrinsic atrial event or causes a paced atrial event. Similarly, the i2i communication protocol may also require the vLP to transmit an i2i event message to the aLP whenever the vLP senses an intrinsic ventricular event or causes a paced ventricular event. The i2i event messages sent between the LPs can be conductive communication messages. That is, conductive communication, which is more energy efficient than radio frequency (RF) communication and inductive communication, may be utilized for the i2i communication. Alternatively, RF or inductive communication may be utilized for the i2i communication that takes place between LPs. The longevity of implantable medical devices (IMDs), such as LPs, is dependent on many factors, including the power consumed by such IMDs to perform i2i communication. In certain dual chamber LP systems, the aLP has a smaller battery than the vLP, and the output impedance for the aLP is lower than that for the vLP. A known dual chamber leadless pacemaker system is disclosed in US 2021/205628 A1.

SUMMARY

The present invention is defined in the independent claims. Further embodiments of the invention are defined in the dependent claims.

An implantable system, according to certain embodiments of the present technology, includes an atrial leadless pacemaker (aLP) configured to be implanted in or on an atrial chamber, and a ventricular leadless pacemaker (vLP) configured to be implanted in or on a ventricular chamber. The aLP is configured to determine an atrial-to-atrial interval (AA interval) for an atrial event, wherein the AA interval comprises a duration between the atrial event and an immediately preceding atrial event. Additionally, the aLP is configured to determine whether or not a variation associated with the AA interval is within a specified tolerance, and based thereon, determine whether or not an atrial event message should be transmitted to the vLP to inform the vLP of the atrial event. The aLP is also configured to abstain from transmitting the atrial event message to the vLP, when there is a determination that the atrial event message should not be transmitted to the vLP, to thereby abstain from informing the vLP of the atrial event. Further, the aLP is configured to transmit the atrial event message to the vLP, when there is a determination that the atrial event message should be transmitted to the vLP, to thereby inform the vLP of the atrial event.

In accordance with certain embodiments, the aLP is configured to: determine that the atrial event message should not be transmitted to the vLP when the AA interval is determined to be within specified tolerance; and determine that the atrial event message should be transmitted to the vLP when the AA interval is not determined to be within specified tolerance.

In accordance with certain embodiments, the aLP is configured to: determine that the atrial event message should not be transmitted to the vLP when the AA interval and an immediately preceding AA interval are each within specified tolerance; and determine that the atrial event message should be transmitted to the vLP when at least one of the AA interval or the immediately preceding AA interval is not within specified tolerance.

In accordance with certain embodiments, the aLP is configured to: determine whether or not a respective atrial event message should be transmitted to the vLP for intrinsic atrial events sensed by the aLP; and determine that a respective atrial event message should be transmitted to the vLP for paced atrial events caused by the aLP.

In accordance with certain embodiments, the aLP is configured to determine whether or not a respective atrial event message should be transmitted to the vLP for intrinsic atrial events sensed by the aLP and for paced atrial events caused by the aLP.

In accordance with certain embodiments, the vLP is configured to: use a ventricular-to-ventricular interval (VV interval) timer to determine whether or not a target ventricular interval has elapsed since a most recent ventricular event has occurred; and deliver pacing stimulation to the ventricular chamber, in response to the VV interval timer expiring without the vLP receiving an atrial event message from the aLP during the target ventricular interval.

In accordance with certain embodiments, the vLP is configured to set the VV interval timer to a ventricular target interval when the VV interval timer is reset. In certain such embodiments, the vLP is configured to determine whether or not instances of the atrial event message were received from the aLP during two consecutive most recent cardiac cycles; and when instances of the atrial event message were received from the aLP during the two consecutive most recent cardiac cycles, update the ventricular target interval based on an interval between when the instances of the atrial event message were received from the aLP during the two consecutive most recent cardiac cycles. In other embodiments, an update to the ventricular target interval is determined by the aLP and transmitted to the vLP within an atrial event message, which eliminates the need for the vLP to determine whether or not instances of the atrial event message were received from the aLP during two consecutive most recent cardiac cycles, and also eliminates the need for the vLP to determine an interval between when the instances of the atrial event message were received from the aLP during the two consecutive most recent cardiac cycles.

In accordance with certain embodiments, the vLP is configured to: use an atrioventricular interval (AV interval) timer to determine when a specified AV interval has elapsed since a most recent atrial event has occurred; start the AV interval timer and cancel the VV interval timer in response to receiving an atrial event message from the aLP; and deliver pacing stimulation to the ventricular chamber in response to the AV interval timer expiring.

In accordance with certain embodiments, the aLP is configured to determine whether or not the variation associated with an AA interval is within the specified tolerance by: determining a running delta indicative of a cumulative difference between each consecutive pair of AA intervals determined since the aLP most recently transmitted the atrial event message to the vLP; and determining that the variation associated with the AA interval is within the specified tolerance when the running delta is within the specified tolerance.

In accordance with certain embodiments, the aLP is configured to determine whether or not the variation associated with an AA interval is within the specified tolerance by: determining a cumulative interval between the AA interval and an AA interval corresponding to when the aLP most recently transmitted the atrial event message to the vLP; determining, based on the cumulative interval and the AA interval, a deviation value indicative of how much AA intervals occurring during the cumulative interval deviate from the AA interval corresponding to when the aLP most recently transmitted the atrial event message to the vLP; and determining that the variation associated with the AA interval is within the specified tolerance when the deviation value is within the specified tolerance.

In accordance with certain embodiments, the aLP is configured to determine whether or not the variation associated with an AA interval is within the specified tolerance by determining a delta between the AA interval and an AA interval corresponding to an immediately preceding atrial event, and determining that the variation associated with the AA interval is within the specified tolerance when the delta is within the specified tolerance.

In accordance with certain embodiments, the aLP abstaining from transmitting the atrial event message to the vLP reduces an amount of power consumed by the aLP compared to when the aLP transmits the atrial event message to the vLP.

In accordance with certain embodiments, the aLP is configured to use conductive communication to transmit the atrial event messages, and other types of messages, to the vLP. In other embodiments, the aLP is configured to use radio frequency (RF) communication or inductive communication when to transmit the atrial event messages, and other types of messages, to the vLP.

Certain embodiments of the present technology are directed to methods for use by a dual chamber leadless pacemaker system including an atrial leadless pacemaker (aLP) implanted in or on an atrial chamber and a ventricular leadless pacemaker (vLP) implanted in or on a ventricular chamber. Such a method can include the aLP determining an atrial-to-atrial interval (AA interval) for an atrial event and determining whether or not a variation associated with the AA interval is within a specified tolerance, wherein the AA interval comprises a duration between the atrial event and an immediately preceding atrial event. The method can also include the aLP determining, based on results of the determining whether or not the variation associated with the AA interval is within the specified tolerance, whether or not an atrial event message should be transmitted to the vLP to inform the vLP of the atrial event. The method can further include the aLP, in response to determining that the atrial event message should not be transmitted to the vLP, abstaining from transmitting the atrial event message to the vLP and thereby abstaining from informing the vLP of the atrial event.

In accordance with certain embodiments, the aLP determining whether or not the atrial event message should be transmitted to the vLP comprises the aLP determining that the atrial event message should not be transmitted to the vLP when the AA interval is determined to be within specified tolerance.

In accordance with certain embodiments, the aLP determining whether or not the atrial event message should be transmitted to the vLP comprises: the aLP determining whether or not a variation associated with an immediately preceding AA interval was within the specified tolerance; the aLP determining that the atrial event message should not be transmitted to the vLP when the AA interval and the immediately preceding AA interval are each within specified tolerance; and the aLP determining that the atrial event message should be transmitted to the vLP when at least one of the AA interval or the immediately preceding AA interval is not within specified tolerance.

In accordance with certain embodiments, the atrial event and the immediately preceding atrial event each comprise an intrinsic atrial event sensed by the aLP. In accordance with other embodiments, the atrial event and the immediately preceding atrial event each comprise either one of an intrinsic atrial event sensed by the aLP or a paced atrial event caused by the aLP.

In accordance with certain embodiments, the method also includes the aLP determining a further AA interval when a further atrial event occurs and determining whether or not a variation associated with the further AA interval is within the specified tolerance, wherein the further AA interval corresponds to a duration between the further atrial event and an immediately preceding atrial event. The method also includes the aLP, in response to determining that the variation associated with the further AA interval is not within the specified tolerance, transmitting an atrial event message to the vLP and thereby informing the vLP of the further atrial event.

In accordance with certain embodiments, the vLP uses a ventricular-to-ventricular interval (VV interval) timer to determine whether or not a target ventricular interval has elapsed since a most recent ventricular event has occurred. In certain such embodiments, the method further comprises the vLP, in response to the VV interval timer expiring without the vLP receiving an atrial event message from the aLP during the target ventricular interval, delivering pacing stimulation to the ventricular chamber. In accordance with certain embodiments, the vLP sets the VV interval timer to a ventricular target interval when the VV interval timer is reset.

In certain such embodiments, where it is the vLP's responsibility to selectively update the target ventricular interval, the vLP, in response to receiving the atrial event message from the aLP, determines whether or not the vLP also received an atrial event message from the aLP during an immediately preceding cardiac cycle and thus received instances of the atrial event message from the aLP during two consecutive most recent cardiac cycles. Such a method can also include the vLP, in response to receiving the instances of the atrial event message from the aLP during the two consecutive most recent cardiac cycles, updating the ventricular target interval based on an interval between when the instances of the atrial event message were received from the aLP during the two consecutive most recent cardiac cycles. In accordance with certain embodiments, the vLP cancels the VV interval timer in response to the vLP receiving the atrial event message from the aLP.

In alternative embodiments, where it is the aLP's responsibility (instead of the vLP's responsibility) to update the target ventricular interval, the atrial event message transmitted by the aLP to the vLP includes an update to the ventricular target interval determined by the aLP.

In accordance with certain embodiments, the vLP uses an atrioventricular interval (AV interval) timer to determine when a specified AV interval has elapsed since a most recent atrial event has occurred. In certain such embodiments, the vLP receives the atrial event message from the aLP, and in response thereto, starts the AV interval timer. In accordance with certain embodiments, the vLP delivers pacing stimulation to the ventricular chamber in response to the AV interval timer expiring.

In accordance with certain embodiments, the aLP determining whether or not the variation associated with the AA interval is within the specified tolerance, comprises the aLP: determining a running delta indicative of a cumulative difference between each consecutive pair of AA intervals determined since the aLP most recently transmitted the atrial event message to the vLP; and determining that the variation associated with the AA interval is within the specified tolerance when the running delta is within the specified tolerance.

In accordance with other embodiments, the aLP determining whether or not the variation associated with the AA interval is within the specified tolerance, comprises the aLP: determining a cumulative interval between the AA interval and an AA interval corresponding to when the aLP most recently transmitted the atrial event message to the vLP; determining, based on the cumulative interval and the AA interval, a deviation value indicative of how much AA intervals occurring during the cumulative interval deviate from the AA interval corresponding to when the aLP most recently transmitted the atrial event message to the vLP; and determining that the variation associated with the AA interval is within the specified tolerance when the deviation value is within the specified tolerance.

In accordance with still other embodiments, the aLP determining whether or not the variation associated with the AA interval is within the specified tolerance, comprises the aLP: determining a delta between the AA interval and an AA interval corresponding to an immediately preceding atrial event; and determining that the variation associated with the AA interval is within the specified tolerance when the delta is within the specified tolerance.

In accordance with certain embodiments, the aLP abstaining from transmitting the atrial event message to the vLP reduces an amount of power consumed by the aLP compared to when the aLP transmits the atrial event message to the vLP.

In accordance with certain embodiments, the aLP uses conductive communication when transmitting the atrial event message, and other types of messages, to the vLP. In other embodiments, the aLP uses radio frequency (RF) communication or inductive communication when transmitting the atrial event message, and other types of messages, to the vLP.

In accordance with certain embodiments, a method includes an aLP sensing intrinsic atrial events and for each intrinsic atrial event that is sensed determining a respective atrial-to-atrial interval (AA interval) that corresponds to a duration between the intrinsic atrial event and an immediately preceding intrinsic atrial event. The method further includes the aLP, based on the determined AA intervals, selectively transmitting and selectively abstaining from transmitting atrial event messages to the vLP. In accordance with certain embodiments, the aLP determines, for each intrinsic atrial event of at least some of the intrinsic atrial events, whether or not a variation associated with the AA interval corresponding to the intrinsic atrial event is within a specified tolerance, and in dependence thereon the aLP determines whether or not the aLP should transmit an atrial event message to the vLP. In certain such embodiments, the aLP abstains from transmitting the atrial event message to the vLP when the AA interval corresponding to an intrinsic atrial event is within the specified tolerance and a further AA interval corresponding to an immediately preceding intrinsic atrial event is also within the specified tolerance.

Certain embodiments of the present technology are directed to an atrial leadless pacemaker (aLP) configured to be implanted in or on an atrial chamber, wherein the aLP comprises a sense circuit, a transmitter, a pair of electrodes, and a controller. The sense circuit is configured to sense a signal indicative of cardiac activity from which atrial events can be detected. The pair of electrodes is configured to deliver stimulation pulses to the atrial chamber. The controller, which is communicatively coupled to the sense circuit and the transmitter, is configured to determine an atrial-to-atrial interval (AA interval) for an atrial event, wherein the AA interval comprises a duration between the atrial event and an immediately preceding atrial event. The controller is also configured to determine whether or not a variation associated with the AA interval is within a specified tolerance, and based thereon, determine whether or not an atrial event message should be transmitted to the vLP to inform the vLP of the atrial event. Additionally, the controller is configured to cause the transmitter to transmit the atrial event message to the vLP, when there is a determination that the atrial event message should be transmitted to the vLP, to thereby inform the vLP of the atrial event. The controller is further configured to abstain from causing the transmitter to transmit the atrial event message to the vLP, when there is a determination that the atrial event message should not be transmitted to the vLP, to thereby abstain from informing the vLP of the atrial event.

In accordance with certain embodiments, the aLP includes one or more pulse generators, at least one of which is used to provide pulses to the pair of electrodes to thereby cause the pair of electrodes to deliver stimulation pulses to the atrial chamber under control of the controller.

In accordance with certain embodiments, the transmitter comprises a conductive communication transmitter including at least one of the one or more pulse generators that provides pulses to the pair of electrodes, under control of the controller, to thereby cause the pair of electrodes to output conductive communication pulses encoded to include the atrial event message. Alternatively, the transmitter can comprise one of a radio frequency (RF) communication transmitter or an inductive communication transmitter, and the atrial event message can be transmitted as an RF signal or an inductive signal.

In accordance with certain embodiments, the sense circuit comprises a sense amplifier coupled to the pair of electrodes, and the signal indicative of cardiac activity comprises an electrogram (EGM) indicative of cardiac electrical activity. In accordance with other embodiments, the sense circuit comprises an accelerometer or a microphone, and the signal indicative of cardiac activity comprises a heart sounds signal.

In accordance with certain embodiments, the aLP comprises a battery that powers the aLP, and power consumption is reduced when the controller abstains from causing the transmitter to transmit the atrial event message to the vLP, compared to when the controller causes the transmitter to transmit the atrial event message to the vLP.

Certain embodiments of the present technology are directed to a ventricular leadless pacemaker (vLP) configured to be implanted in or on a ventricular chamber, wherein the vLP comprises a sense circuit, a receiver, a pulse generator, a pair of electrodes, and a controller. The sense circuit is configured to sense a signal indicative of cardiac activity from which ventricular events can be detected. The pair of electrodes is configured to deliver stimulation pulses output by the pulse generator to the ventricular chamber. The controller is communicatively coupled to the sense circuit, the pulse generator, and the receiver. An atrioventricular interval (AV interval) timer is used to determine whether a specified AV interval has elapsed since a most recent atrial event, and a ventricular-to-ventricular interval (VV interval) timer is used to determine whether a target ventricular interval has elapsed since a most recent ventricular event. At least one of the AV interval timer and the VV interval timer can be implemented by the controller, but that need not be the case. The controller of the vLP is configured to start the AV interval timer when an atrial event message is received by the vLP from an atrial leadless pacemaker (aLP) configured to be implanted in or on an atrial chamber. The controller of the vLP is also configured to cause to the pulse generator to provide a stimulation pulse to the pair of electrodes, when the AV interval timer expires, to thereby cause the ventricular chamber to be paced by the vLP at approximately the AV interval following an immediately preceding atrial event. Additionally, the controller of the vLP is configured to set the VV interval timer to a ventricular target interval when the VV interval timer is reset, start the VV interval timer in response to a ventricular event, and cause to the pulse generator to provide a stimulation pulse to the pair of electrodes and cause the VV interval timer to be reset, when the VV interval timer expires without the receiver receiving the atrial event message from the aLP, to thereby cause the ventricular chamber to be paced at approximately the ventricular target interval following an immediately preceding ventricular event. Additionally, the controller of the vLP is configured to cancel the VV interval timer, when the vLP receives the atrial event message from the aLP.

In certain embodiments, where it is the vLP's responsibility to selectively update the ventricular target interval, the controller of the vLP is also configured to determine whether the atrial event message was also received from the aLP during an immediately preceding cardiac cycle and thus instances of the atrial event message were received from the aLP during two consecutive most recent cardiac cycles. Further, when the instances of the atrial event message were received from the aLP during two consecutive most recent cardiac cycles, the controller of the vLP is configured to update the ventricular target interval based on an interval between when the instances of the atrial event message were received from the aLP during the two consecutive most recent cardiac cycles.

In alternative embodiments, where it is the aLP's responsibility (instead of the vLP's responsibility) to update the target ventricular interval, the atrial event message transmitted by the aLP to the vLP, and received by the vLP, includes an update to the ventricular target interval that was determined by the aLP. In such alternative embodiments, the vLP does not need to determine updates to the ventricular target interval from two consecutive atrial event messages received from aLP. Instead the aLP can explicitly inform the vLP of a new value for (i.e., an update to) the ventricular target interval via a single atrial event message. In certain such embodiments, the new value for (i.e., an update to) the ventricular target interval, which is determined by the aLP, is equal to the AA interval most recently determined by the aLP.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

BRIEF DESCRIPTION OF THE DRAWINGS

Embodiments of the present technology relating to both structure and method of operation may best be understood by referring to the following description and accompanying drawings, in which similar reference characters denote similar elements throughout the several views:

FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 is a timing diagram demonstrating one embodiment of implant to implant (i2i) communication for a paced event.
FIG. 5 is a timing diagram demonstrating one embodiment of i2i communication for a sensed event.
FIG. 6 is an example timing diagram demonstrating that when an atrial leadless pacemaker (aLP) detects a series of intrinsic atrial events, a ventricular leadless pacemaker (vLP) can track those atrial events (via receipt of associated i2i atrial event messages transmitted by the aLP to the vLP) and pace a ventricular chamber after defined AV intervals.
FIGS. 7A-7D are high level flow diagrams used to summarize various methods of the present technology that are performed by an aLP, of a dual chamber leadless pacemaker system that also includes a vLP, to enable the aLP to selectively abstain from transmitting atrial event messages to the vLP so that the aLP conserves power used for i2i communication and thereby increases its longevity.
FIGS. 8 and 9 are high level flow diagrams used to summarize various different manners that an aLP can perform certain steps introduced in FIGS. 7A-7D which relate to the aLP determining whether or not a variability associated with an AA interval is within a specified tolerance, wherein the results of such a determination are used by the aLP to determine whether or not the aLP should abstain from transmitting an atrial event messages to the vLP.
FIG. 10 is a high level flow diagram used to summarize certain methods of the present technology that are performed by a vLP, of a dual chamber leadless pacemaker system that also includes an aLP, that enable the vLP to rely on its VV interval timer and its AV interval timer to know whether and when it should pace a ventricular chamber, and selectively redefine a ventricular target interval that is used by the VV interval timer.
FIG. 11 shows a block diagram of one embodiment of an IMD (e.g., LP) that is implanted into a patient as part of the implantable cardiac system in accordance with certain embodiments herein.

DETAILED DESCRIPTION

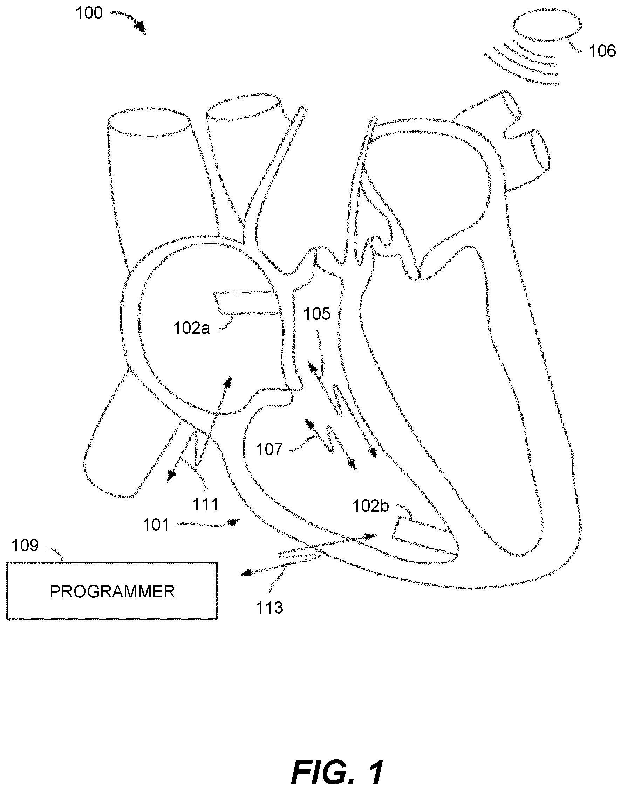

Certain embodiments of the present technology generally relate to dual chamber leadless pacemaker systems, leadless pacemakers thereof, and methods for use by dual chamber leadless pacemaker systems and leadless pacemakers thereof. Such a dual chamber leadless pacemaker system, as described below, include an atrial leadless pacemaker (aLP) implanted in or on an atrial chamber and a ventricular leadless pacemaker (vLP) implanted in or on a ventricular chamber. As will be described in additional details below, certain embodiments enable the aLP to selectively abstain from transmitting atrial event messages to the vLP, to thereby conserve power used by the aLP and increase it longevity. Other embodiments enable the vLP to operate properly when the vLP does not receive atrial event messages from the aLP, because the aLP had abstained from transmitting atrial event messages to the vLP. Before providing additional details of the specific embodiments of the present technology mentioned above, an exemplary dual chamber leadless pacemaker system in which embodiments of the present technology can be used will first be described with reference to FIGS. 1-5. A leadless pacemaker can also be referred to herein as a leadless cardiac pacemaker, or more succinctly as an LP. Where a cardiac pacing system includes a subcutaneous-ICD (S-ICD), the S-ICD may perform certain sensing operations and may communicate with one or more LPs by sending and/or receiving messages to and/or from one or more LPs, as can be appreciated from the below discussion. Where a cardiac pacing system includes a programmer, the programmer may be used to program one or more IMDs (e.g., LPs), download information to one or more IMDs, and/or upload information from one or more IMDs, as can be appreciated from the below description.

FIG. 1 illustrates a system 100 that is configured to be implanted in a heart 101. The system 100 includes two or more leadless pacemakers (LPs) 102a and 102b located in different chambers of the heart. LP 102a is located in a right atrium, while LP 102b is located in a right ventricle. LPs 102a and 102b communicate with one another to inform one another of various local physiologic activities, such as local intrinsic events, local paced events and the like. LPs 102a and 102b may be constructed in a similar manner, but operate differently based upon which chamber LP 102a or 102b is located. The LPs 102a and 102b may sometimes be referred to collectively herein as the LPs 102, or individually as an LP 102.

In certain embodiments, LPs 102a and 102b communicate with one another, and/or with an ICD 106, by conductive communication through the same electrodes that are used for sensing and/or delivery of pacing therapy. The LPs 102a and 102b may also be able to use conductive communication to communicate with an external device, e.g., a programmer 109, having electrodes placed on the skin of a patient within with the LPs 102a and 102b are implanted. While not shown (and not preferred, since it would increase the size and power consumption of the LPs 102a and 102b), the LPs 102a and 102b can potentially include an antenna and/or telemetry coil that would enable them to communicate with one another, the ICD 106 and/or an external device using RF or inductive communication. While only two LPs are shown in FIG. 1, it is possible that more than two LPs can be implanted in a patient. For example, to provide for bi-ventricular pacing and/or cardiac resynchronization therapy (CRT), in addition to having LPs implanted in the right atrial (RA) chamber and the right ventricular (RV) chamber, a further LP can be implanted in the left ventricular (LV) chamber.

In some embodiments, one or more LP 102a can be co-implanted with the ICD 106. Each LP 102a uses two or more electrodes located within, on, or within a few centimeters of the housing of the pacemaker, for pacing and sensing at the cardiac chamber, for bidirectional communication with one another, with the programmer 109, and the ICD 106.

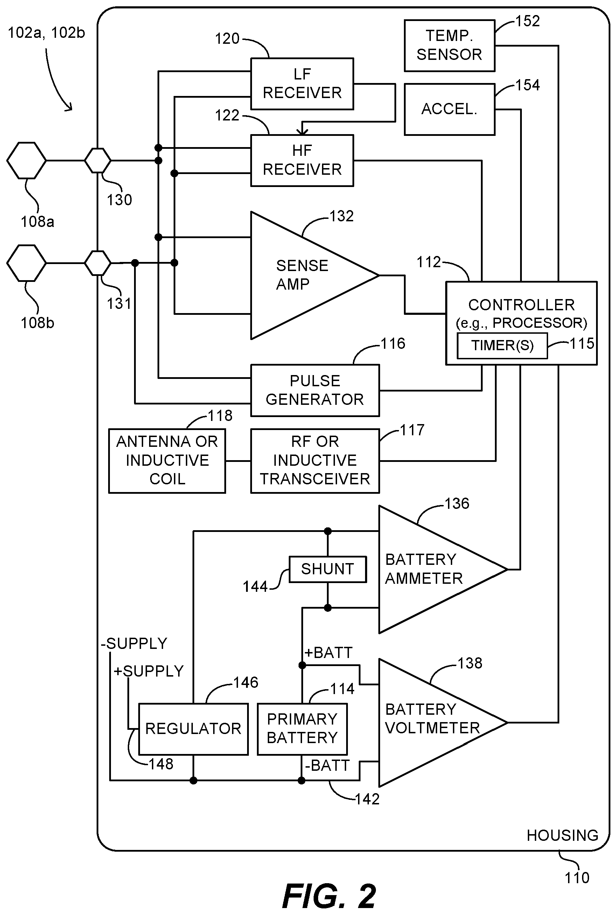

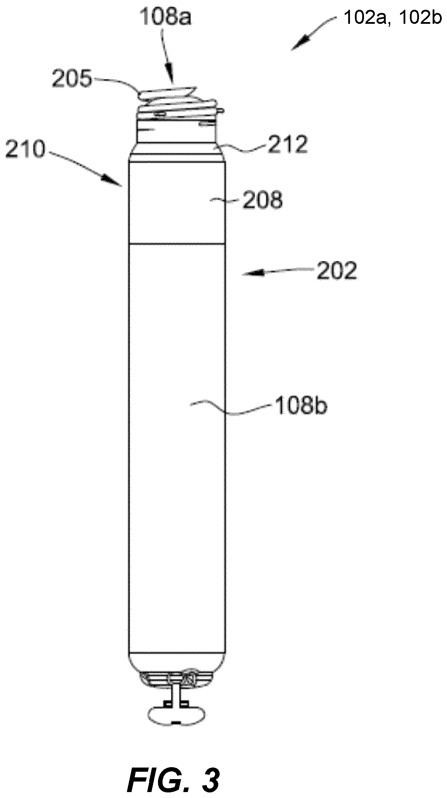

Referring to FIG. 2, a block diagram shows an embodiment for portions of the electronics within LPs 102a, 102b configured to provide conductive communication through the sensing/pacing electrode. One or more of LPs 102a and 102b include at least two leadless electrodes configured for delivering cardiac pacing pulses, sensing evoked and/or natural cardiac electrical signals, and uni-directional or bi-directional communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation.

In FIG. 2, each of the LPs 102a, 102b is shown as including first and second receivers 120 and 122 that collectively define separate first and second communication channels 105 and 107 (FIG. 1), (among other things) between LPs 102a and 102b. Although first and second receivers 120 and 122 are depicted, in other embodiments, each LP 102a, 102b may only include the first receiver 120, or may include additional receivers other than first and second receivers 120 and 122. As will be described in additional detail below, the pulse generator 116 can function as a transmitter that transmits i2i communication signals using the electrodes 108. In certain embodiments, LPs 102a and 102b may communicate over more than just first and second communication channels 105 and 107. In certain embodiments, LPs 102a and 102b may communicate over one common communication channel 105. More specifically, LPs 102a and 102b can communicate conductively over a common physical channel via the same electrodes 108 that are also used to deliver pacing pulses. Usage of the electrodes 108 for communication enables the one or more LPs 102a and 102b to perform antenna-less and telemetry coil-less communication.

The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 100 kHz) and the receiver 122 is configured to monitor for one or more signals within a relatively high frequency range (e.g., above 100 kHz). In certain embodiments, the receiver 120 (and more specifically, at least a portion thereof) is always enabled and monitoring for a wakeup notice, which can simply be a wakeup pulse, within a specific low frequency range (e.g., between 1 kHz and 100 kHz); and the receiver 122 is selectively enabled by the receiver 120. The receiver 120 is configured to consume less power than the receiver 122 when both the first and second receivers are enabled. Accordingly, the receiver 120 can also be referred to as a low power receiver 120, and the receiver 122 can also be referred to as a high power receiver 122. The low power receiver 120 is incapable of receiving signals within the relatively high frequency range (e.g., above 100 kHz), but consumes significantly less power than the high power receiver 122. This way the low power receiver 120 is capable of always monitoring for a wakeup notice without significantly depleting the battery (e.g., 114) of the LP. In accordance with certain embodiments, the high power receiver 122 is selectively enabled by the low power receiver 120, in response to the low power receiver 120 receiving a wakeup notice, so that the high power receiver 122 can receive the higher frequency signals, and thereby handle higher data throughput needed for effective i2i communication without unnecessarily and rapidly depleting the battery of the LP (which the high power receiver 122 may do if it were always enabled).

In accordance with certain embodiments, when one of the LPs 102a and 102b senses an intrinsic event or delivers a paced event, the corresponding LP 102a, 102b transmits an implant event message to the other LP 102a, 102b. Where an event message originates from an LP (e.g., 102a) implanted in or on an atrial chamber (e.g., the right atrial chamber), the event message can be referred to more specifically as an atrial event message. Where an event message originates from an LP (e.g., LP 102b) implanted in or on a ventricular chamber (e.g., the right ventricular chamber), the event message can be referred to more specifically as a ventricular event message. For example, when an atrial LP 102a senses/paces an atrial event, the atrial LP 102a transmits an atrial event message including an event marker indicative of a nature of the event (e.g., intrinsic/sensed atrial event, paced atrial event). When a ventricular LP 102b senses/paces a ventricular event, the ventricular LP 102b transmits a ventricular event message including an event marker indicative of a nature of the event (e.g., intrinsic/sensed ventricular event, paced ventricular event). In certain embodiments, each LP 102a, 102b transmits a paced event message to the other LP 102a, 102b preceding delivery of an actual pace pulse so that the remote LP can blank its sense inputs in anticipation of that remote pace pulse (to prevent inappropriate crosstalk sensing). In alternative embodiments, each LP 102a, 102b transmits a paced event message to the other LP 102a, 102b following delivery of actual pace pulse, and the remote LP relies on an alternative technique for avoiding crosstalk sensing.

The implant event messages may be formatted in various manners. As one example, each event message may include a leading trigger pulse (also referred to as an LP wakeup notice, wakeup pulse or wakeup signal) followed by an event marker. The notice trigger pulse (also referred to as the wakeup notice, wakeup pulse or wakeup signal) is transmitted over a first channel (e.g., with a pulse duration of approximately 10$\mu$s to approximately 1ms and/or within a fundamental frequency range of approximately 1kHz to approximately 100kHz). The notice trigger pulse indicates that an event marker is about to be transmitted over a second channel (e.g., within a higher frequency range). The event marker can then be transmitted over the second channel.

The event markers may include data indicative of one or more events (e.g., a sensed intrinsic atrial activation for an atrial located LP, a sensed intrinsic ventricular activation for a ventricular located LP). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP (or other IMD) that receives any i2i communication signal from another LP (or other IMD) or from an external device may transmit a receive acknowledgement indicating that the receiving LP (or other IMD) received the i2i communication signal. In certain embodiments, where an IMD expects to receive an i2i communication signal within a window, and fails to receive the i2i communication signal within the window, the IMD may transmit a failure-to-receive acknowledgement indicating that the receiving IMD failed to receive the i2i communication signal. Other variations are also possible and within the scope of the embodiments described herein.

The event messages enable the LPs 102a, 102b to deliver synchronized therapy and additional supportive features (e.g., measurements, etc.). To maintain synchronous therapy, each of the LPs 102a and 102b is made aware (through the event messages) when an event occurs in the chamber containing the other LP 102a, 102b. Some embodiments described herein provide efficient and reliable processes to maintain synchronization between LPs 102a and 102b without maintaining continuous communication between LPs 102a and 102b. In accordance with certain embodiments herein, low power event messages/signaling may be maintained between LPs 102a and 102b synchronously or asynchronously.

For synchronous event signaling, LPs 102a and 102b may maintain synchronization and regularly communicate at a specific interval. Synchronous event signaling allows the transmitter and receivers in each LP 102a, 102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20$\mu$s, where the Tx/Rx time slots occur periodically (e.g., every 10-20ms).

LPs 102a and 102b may lose synchronization, even in a synchronous event signaling scheme. As explained herein, features may be included in LPs 102a and 102b to maintain device synchronization, and when synchronization is lost, LPs 102a and 102b undergo operations to recover synchronization. Also, synchronous event messages/signaling may introduce a delay between transmissions which causes a reaction lag at the receiving LP 102a, 102b. Accordingly, features may be implemented to account for the reaction lag.

During asynchronous event signaling, LPs 102a and 102b do not maintain communication synchronization. During asynchronous event signaling, one or more of receivers 120 and 122 of LPs 102a and 102b may be "always on" (always awake) to search for incoming transmissions. However, maintaining LP receivers 120, 122 in an "always on" (always awake) state presents challenges as the received signal level often is low due to high channel attenuation caused by the patient's anatomy. Further, maintaining the receivers awake will deplete the battery 114 more quickly than may be desirable.

The asynchronous event signaling methods avoid risks associated with losing synchronization between devices. However, the asynchronous event signaling methods utilize additional receiver current between transmissions. For purposes of illustration only, a non-limiting example is described hereafter. For example, the channel attenuation may be estimated to have a gain of 1/500 to 1/10000. A gain factor may be 1/1000th. Transmit current is a design factor in addition to receiver current. As an example, the system may allocate one-half of the implant communication current budget to the transmitter (e.g., 0.5μA for each transmitter). When LP 102a, 102b maintains a transmitter in a continuous on-state and the electrode load is 500 ohms, a transmitted voltage may be 2.5V. When an event signal is transmitted at 2.5V, the event signal is attenuated as it propagates and would appear at LP 102a, 102b receiver as an amplitude of approximately 0.25mV.

To overcome the foregoing receive power limit, a pulsed transmission scheme may be utilized in which communication transmissions occur correlated with an event. By way of example, the pulsed transmission scheme may be simplified such that each transmission constitutes a single pulse of a select amplitude and width.

In accordance with certain embodiments herein, LPs 102a and 102b may utilize multi-stage receivers that implement a staged receiver wakeup scheme in order to improve reliability yet remain power efficient. Each of LPs 102a and 102b may include first and second receivers 120 and 122 that operate with different first and second activation protocols and different first and second receive channels. For example, first receiver 120 may be assigned a first activation protocol that is "always on" (also referred to as always awake) and that listens over a first receive channel that has a lower fundamental frequency range/pulse duration (e.g., 1kHz to 100kHz / 10μs to approximately 1ms) as compared to the fundamental frequency range (e.g., greater than 100kHz / less than 10μs per pulse) assigned to the second receive channel.

In accordance with certain embodiments, the first receiver 120 may maintain the first channel active (awake) at all times (including when the second channel is inactive (asleep)) in order to listen for messages from a remote LP. The second receiver 122 may be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP). The terms active, awake and enabled are used interchangeably herein.

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). For example, the controller 112 of the LP 102b can be used to implement one or more timers 115, including but not limited to, an AV interval timer and a ventricular-to-ventricular interval (VV interval) timer. The controller 112 of the LP 102a can similarly be used to implement one or more timers 115 that may be used by the LP 102a. Such timing control circuitry may also be used for the timing of refractory periods, blanking intervals, noise detection windows, evoked response windows, alert intervals, marker channel timing, and so on.

The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, a LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or programmer 109.

In accordance with certain embodiments herein, programmer 109 may communicate over a programmer-to-LP channel, with LP 102a, 102b utilizing the same communication scheme. The external programmer may listen to the event message transmitted between LP 102a, 102b and synchronize programmer to implant communication such that programmer 109 does not transmit communication signals 113 until after an implant to implant messaging sequence is completed.

In accordance with certain embodiments, LP 102a, 102b may combine transmit operations with therapy. The transmit event marker may be configured to have similar characteristics in amplitude and pulse-width to a pacing pulse and LP 102a, 102b

While not shown, a communication capacitor can be provided in LP 102a, 102b. The communication capacitor may be used to transmit event signals having higher voltage for the event message pulses to improve communication, such as when the LPs 102a and 102b experience difficulty sensing event messages. The high voltage event signaling may be used for implants with high signal attenuation or in the case of a retry for an ARQ (automatic repeat request) handshaking scheme.

In some embodiments, the individual LP 102a can comprise a hermetic housing 110 configured for placement on or attachment to the inside or outside of a cardiac chamber and at least two leadless electrodes 108 proximal to the housing 110 and configured for bidirectional communication with at least one other device 106 within or outside the body.

FIG. 2 depicts a single LP 102a (or 102b) and shows the LP's functional elements substantially enclosed in a hermetic housing 110. The LP 102a (or 102b) has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example a battery current monitor 136 and a battery voltage monitor 138, and can contain circuits for controlling operations in a predetermined manner.

The electrodes 108 can be configured to communicate bidirectionally among the multiple leadless cardiac pacemakers and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more leadless cardiac pacemakers 102a and/or the ICD 106 and transmit data including designated codes for events detected or created by an individual pacemaker. Individual pacemakers can be configured to issue a unique code corresponding to an event type and a location of the sending pacemaker.

Moreover, information communicated on the incoming channel can also include an event message from another leadless cardiac pacemaker signifying that the other leadless cardiac pacemaker has sensed a heartbeat or has delivered a pacing pulse, and identifies the location of the other pacemaker. For example, LP 102b may receive and relay an event message from LP 102a to the programmer. Similarly, information communicated on the outgoing channel can also include a message to another leadless cardiac pacemaker or pacemakers, or to the ICD, that the sending leadless cardiac pacemaker has sensed a heartbeat or has delivered a pacing pulse at the location of the sending pacemaker.

Referring again to FIGS. 1 and 2, the cardiac pacing system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the one or more LPs 102a, 102b configured for leadless intercommunication by information conduction through body tissue and/or wireless transmission between transmitters and receivers in accordance with the discussed herein.

In a further embodiment, a cardiac pacing system 100 comprises at least one LP 102a, 102b configured for implantation in electrical contact with a cardiac chamber and configured to perform cardiac pacing functions in combination with the co-implanted ICD 106. The leadless cardiac pacemaker or pacemakers 102a comprise at least two leadless electrodes 108 configured for delivering cardiac pacing pulses, sensing evoked and/or natural cardiac electrical signals, and transmitting information to the co-implanted ICD 106.

As shown in the illustrative embodiments, a leadless cardiac pacemaker 102a, 102b can comprise two or more leadless electrodes 108 configured for delivering cardiac pacing pulses, sensing evoked and/or natural cardiac electrical signals, and bidirectionally communicating with the co-implanted ICD 106.

LP 102a, 102b can be configured for operation in a particular location and a particular functionality at manufacture and/or at programming by an external programmer. Bidirectional communication among the multiple leadless cardiac pacemakers can be arranged to communicate notification of a sensed heartbeat or delivered pacing pulse event and encoding type and location of the event to another implanted pacemaker or pacemakers. LP 102a, 102b receiving the communication decode the information and respond depending on location of the receiving pacemaker and predetermined system functionality.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in which the pacemaker is implanted (and thus with which the LP is interacting; e.g., pacing and/or sensing). Some non-limiting examples include sensing sensitivity, an evoked response algorithm, use of AF suppression in a local chamber, blanking & refractory periods, etc. Accordingly, each LP needs to know an identity of the chamber in which the LP is implanted, and processes may be implemented to automatically identify a local chamber associated with each LP.

Also shown in FIG. 2, the primary battery 114 has positive terminal 140 and negative terminal 142. Current from the positive terminal 140 of primary battery 114 flows through a shunt 144 to a regulator circuit 146 to create a positive voltage supply 148 suitable for powering the remaining circuitry of the pacemaker 102. The shunt 144 enables the battery current monitor 136 to provide the controller (e.g., processor) 112 with an indication of battery current drain and indirectly of device health. The illustrative power supply can be a primary battery 114.

Referring to FIG. 2, the LP is shown as including a temperature sensor 152. The temperature sensor can be any one of various different types of well-known temperature sensors, or can be a future developed temperature sensor. For one example, the temperature sensor 152 can be a thermistor, a thermocouple, a resistance thermometer, or a silicon bandgap temperature sensor, but is not limited thereto. Regardless of how the temperature sensor 152 is implemented, it is preferably that the temperature sensed by the sensor is provided to the controller 112 as a digital signal indicative of the blood temperature of the patient within which the LP is implanted. The temperature sensor 152 can be hermetically sealed within the housing 110, but that need not be the case. The temperature sensor 152 can be used in various manners. For example, the temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the temperature sensor 152.

Referring to FIG. 2, the LP is also shown as including an accelerometer 154 which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the accelerometer 154 can be used to detect an activity level of the patient to adjust a pacing rate, i.e., for use in rate responsive pacing. It would also be possible to use outputs of both the accelerometer 154 and the temperature sensor 152 to monitor the activity level of a patient. Alternatively, or additionally, a patient's activity level can be monitored based on their heart rate, as detected from an electrogram (EGM) sensed using the electrodes 108, and/or sensed using a plethysmography signal obtained using a plethysmography sensor (not shown) or a heart sound sensor (not shown), but not limited thereto. One or more signals produced and output by the accelerometer 154 may be analyzed with respect to frequency content, energy, duration, amplitude and/or other characteristics. Such signals may or may not be amplified and/or filtered prior to being analyzed. For example, filtering may be performed using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain. The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. Other variations are also possible, and within the scope of embodiments of the present technology. In accordance with certain embodiments of the present technology, described in additional detail below, a sensor signal produced by the accelerometer 154 of an LP implanted in or on a cardiac chamber can be used to detect mechanical cardiac activity associated with another cardiac chamber.

The controller of the LP 102a can detect intrinsic atrial events from an EGM that is sensed by the LP 102a. Alternatively, or additionally, the controller of the LP 102a can detect intrinsic atrial events from one or more signals sensed by the accelerometer 154 thereof and/or from a heart sounds signal sensed by a microphone (not shown) of the LP 102a. The controller of the LP 102b can detect intrinsic ventricle events from an EGM that is sensed by the LP 102b. Alternatively, or additionally, the controller of the LP 102b can detect intrinsic ventricular events from one or more signals sensed by the accelerometer 154 thereof and/or from a heart sounds signal sensed by a microphone (not shown) of the LP 102b. In certain embodiments, each of the LPs 102 includes only one of the temperature sensor 152 and the accelerometer 154.

In various embodiments, LP 102a, 102b can manage power consumption to draw limited power from the battery, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 in one leadless cardiac pacemaker 102a can access signals on the electrodes 108 and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

Instead of or in addition to the LP 102 utilizing conductive communication to communicate with another LP, another type of IMD, and/or an external device (e.g., 109), the LP 102 can include an RF or inductive transceiver 117 that is coupled to the controller 112, and the RF or inductive transceiver 117 can be coupled to an antenna or inductive coil 118, to thereby enable the LP 102 to utilize RF communication and/or inductive communication to communicate with another LP, another type of IMD, and/or an external device (e.g., 109). In other words, the communication performed by the LP 102 can be conductive communication, RF communication, or inductive communication, or any combination thereof.

FIG. 2 shows an LP 102a, 102b. The LP can include a hermetic housing 202 with electrodes 108a and 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 2.

The housing can also include an electronics compartment 210 within the housing that contains the electronic components necessary for operation of the pacemaker, including, e.g., a pulse generator, receiver, a battery, and a processor for operation. The hermetic housing 202 can be adapted to be implanted on or in a human heart, and can be cylindrically shaped, rectangular, spherical, or any other appropriate shapes, for example.

The housing can comprise a conductive, biocompatible, inert, and anodically safe material such as titanium, 316L stainless steel, or other similar materials. The housing can further comprise an insulator disposed on the conductive material to separate electrodes 108a and 108b. The insulator can be an insulative coating on a portion of the housing between the electrodes, and can comprise materials such as silicone, polyurethane, parylene, or another biocompatible electrical insulator commonly used for implantable medical devices. In the embodiment of FIG. 2, a single insulator 208 is disposed along the portion of the housing between electrodes 108a and 108b. In some embodiments, the housing itself can comprise an insulator instead of a conductor, such as an alumina ceramic or other similar materials, and the electrodes can be disposed upon the housing.

As shown in FIG. 2, the pacemaker can further include a header assembly 212 to isolate 108a and 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 2, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 2) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

Implant-to-Implant Event Messaging

LPs 102a and 102b can utilize implant-to-implant (i2i) communication through event messages to coordinate operation with one another in various manners. The terms i2i communication, i2i event messages, and i2i event markers are used interchangeably herein to refer to event related messages and IMD/IMD operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall be referred to as "vLP" and the atrial LP 102a shall be referred to as "aLP". The LP 102 that is designated as the master device (e.g. vLP) may implement all or most dual-chamber diagnostic and therapy determination algorithms. For purposes of the following illustration, it is assumed that the vLP is a "master" device, while the aLP is a "slave" device. Alternatively, the aLP may be designated as the master device, while the vLP may be designated as the slave device. The master device orchestrates most or all decision-making and timing determinations (including, for example, rate-response changes).

In accordance with certain embodiments, methods are provided for coordinating operation between first and second leadless pacemakers (LPs) configured to be implanted entirely within first and second chambers of the heart. A method transmits an event marker through conductive communication through electrodes located along a housing of the first LP, the event marker indicative of one of a local paced or sensed event. The method detects, over a sensing channel, the event marker at the second LP. The method identifies the event marker at the second LP based on a predetermined pattern configured to indicate that an event of interest has occurred in a remote chamber. In response to the identifying operation, the method initiates a related action in the second LP.

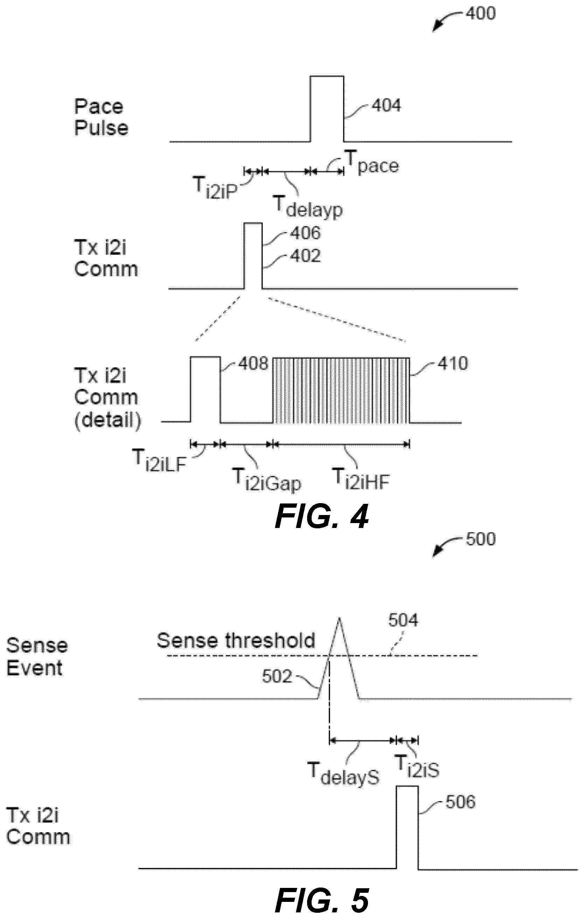

FIG. 4 is a timing diagram 400 demonstrating one example of an i2i communication for a paced event. The i2i communication may be transmitted, for example, from LP 102a to LP 102b. As shown in FIG. 4, in this embodiment, an i2i transmission 402 is sent prior to delivery of a pace pulse 404 by the transmitting LP (e.g., LP 102). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period $T_{i2iLF}$, and high frequency pulse train 410 lasts for a period $T_{i2iHF}$. The end of low frequency pulse 408 and the beginning of high frequency pulse train 410 are separated by a gap period, $T_{i2iGap}$.

As shown in FIG. 4, the i2i transmission 402 lasts for a period Ti2iP, and pace pulse 404 lasts for a period Tpace. The end of i2i transmission 402 and the beginning of pace pulse 404 are separated by a delay period, TdelayP. The delay period may be, for example, between approximately 0.0 and 10.0 milliseconds (ms), particularly between approximately 0.1 ms and 2.0 ms, and more particularly approximately 1.0 ms. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a detects the sensed event when a sensed intrinsic activation 502 crosses a sense threshold 504. A predetermined delay period, $T_{delayS}$, after the detection, the transmitting LP transmits an i2i transmission 506 that lasts a predetermined period $T_{i2iS}$. The delay period may be, for example, between approximately 0.0 and 10.0 milliseconds (ms), particularly between approximately 0.1 ms and 2.0 ms, and more particularly approximately 1.0 ms.

As with i2i transmission 402, i2i transmission 506 may include an envelope that may include one or more individual pulses. For example, similar to envelope 406, the envelope of i2i transmission 506 may include a low frequency pulse followed by a high frequency pulse train.

Optionally, wherein the first LP is located in an atrium and the second LP is located in a ventricle, the first LP produces an AS/AP event marker to indicate that an atrial sensed (AS) event has occurred or an atrial paced (AP) event has occurred or will occur in the immediate future. For example, the AS and AP event markers may be transmitted following the corresponding AS or AP event. Alternatively, the first LP may transmit the AP event marker slightly prior to delivering an atrial pacing pulse. In certain embodiments, wherein the first LP is located in an atrium and the second LP is located in a ventricle, the second LP initiates an atrioventricular (AV) interval after receiving an AS or AP event marker from the first LP; and initiates a post atrial ventricular blanking (PAVB) interval after receiving an AP event marker from the first LP.

In accordance with some embodiments, communication and synchronization between the aLP and vLP is implemented via conducted communication of markers/commands in the event messages (per i2i communication protocol). As explained above, conducted communication represents event messages transmitted from the sensing/pacing electrodes at frequencies outside the RF or Wi-Fi frequency range. Alternatively, the event messages may be conveyed over communication channels operating in the RF or Wi-Fi frequency range. The figures and corresponding description below illustrate non-limiting examples of markers that may be transmitted in event messages. The figures and corresponding description below also include the description of the markers and examples of results that occur in the LP that receives the event message. Table 1 represents exemplary event markers sent from the aLP to the vLP, while Table 2 represents exemplary event markers sent from the vLP to the aLP. In the master/slave configuration, AS event markers are sent from the aLP each time that an atrial event is sensed outside of the post ventricular atrial blanking (PVAB) interval or some other alternatively-defined atrial blanking period. The AP event markers are sent from the aLP each time that the aLP delivers a pacing pulse in the atrium. The aLP may restrict transmission of AS markers, whereby the aLP transmits AS event markers when atrial events are sensed both outside of the PVAB interval and outside the post ventricular atrial refractory period (PVARP) or some other alternatively-defined atrial refractory period. Alternatively, the aLP may not restrict transmission of AS event markers based on the PVARP, but instead transmit the AS event marker every time an atrial event is sensed.

Table 1

| "A2V" Markers / Commands (*i.e.*, from aLP to vLP) | | |
|---|---|---|
| *Marker* | *Description* | *Result in vLP* |
| AS | Notification of a sensed event in atrium (if not in PVAB or PVARP) | • Initiate AV interval (if not in PVAB or PVARP) |

(continued)

| "A2V" Markers / Commands (*i.e.*, from aLP to vLP) | | |
|---|---|---|
| Marker | Description | Result in vLP |
| AP | Notification of a paced event in atrium | • Initiate PAVB<br>• Initiate AV interval (if not in PVARP) |

As shown in Table 1, when an aLP transmits an event message that includes an AS event marker (indicating that the aLP sensed an intrinsic atrial event), the vLP initiates an AV interval timer. If the aLP transmits an AS event marker for all sensed events, then the vLP would preferably first determine that a PVAB or PVARP interval is not active before initiating an AV interval timer. If however the aLP transmits an AS event marker only when an intrinsic signal is sensed outside of a PVAB or PVARP interval, then the vLP could initiate the AV interval timer upon receiving an AS event marker without first checking the PVAB or PVARP status. When the aLP transmits an AP event marker (indicating that the aLP delivered or is about to deliver a pace pulse to the atrium), the vLP initiates a PVAB timer and an AV interval timer, provided that a PVARP interval is not active. The vLP may also blank its sense amplifiers to prevent possible crosstalk sensing of the remote pace pulse delivered by the aLP.

Table 2

| "V2A" Markers / Commands (*i.e.*, from vLP to aLP) | | |
|---|---|---|
| Marker | Description | Result in aLP |
| VS | Notification of a sensed event in ventricle | • Initiate PVARP |
| VP | Notification of a paced event in ventricle | • Initiate PVAB<br>• Initiate PVARP |
| AP | Command to deliver immediate pace pulse in atrium | • Deliver immediate pace pulse to atrium |

As shown in Table 2, when the vLP senses a ventricular event, the vLP transmits an event message including a VS event marker, in response to which the aLP may initiate a PVARP interval timer. When the vLP delivers or is about to deliver a pace pulse in the ventricle, the vLP transmits VP event marker. When the aLP receives the VP event marker, the aLP initiates the PVAB interval timer and also the PVARP interval timer. The aLP may also blank its sense amplifiers to prevent possible crosstalk sensing of the remote pace pulse delivered by the vLP. The vLP may also transmit an event message containing an AP command marker to command the aLP to deliver an immediate pacing pulse in the atrium upon receipt of the command without delay.

The foregoing event markers are examples of a subset of markers that may be used to enable the aLP and vLP to maintain full dual chamber functionality. In one embodiment, the vLP may perform all dual-chamber algorithms, while the aLP may perform atrial-based hardware-related functions, such as PVAB, implemented locally within the aLP. In this embodiment, the aLP is effectively treated as a remote 'wireless' atrial pace/sense electrode. In another embodiment, the vLP may perform most but not all dual-chamber algorithms, while the aLP may perform a subset of diagnostic and therapeutic algorithms. In an alternative embodiment, vLP and aLP may equally perform diagnostic and therapeutic algorithms. In certain embodiments, decision responsibilities may be partitioned separately to one of the aLP or vLP. In other embodiments, decision responsibilities may involve joint inputs and responsibilities.

In an embodiment, ventricular-based pace and sense functionalities are not dependent on any i2i communication, in order to provide safer therapy. For example, in the event that LP to LP (i2i) communication is lost (prolonged or transient), the system 100 may automatically revert to safe ventricular-based pace/sense functionalities as the vLP device is running all of the necessary algorithms to independently achieve these functionalities. For example, the vLP may revert to a VVI mode as the vLP does not depend on i2i communication to perform ventricular pace/sense activities. Once i2i communication is restored, the system 100 can automatically resume dual-chamber functionalities.

Messages that are transmitted between LPs (e.g., the aLP and the vLP) can be referred to herein generally as i2i messages, since they are implant-to-implant messages. As noted above, such messages can include event markers that enable one LP to inform the other LP of a paced event or a sensed event. For example, in certain embodiments, whenever the aLP senses an atrial event or paces the right atrium, the aLP will transmit an i2i message to the vLP to inform the vLP of the sensed or paced event in the atrium. In response to receiving such an i2i message, the vLP may start one or more timers that enable the vLP to sense or pace in the right ventricle. Similarly, the vLP may transmit an i2i message to the aLP whenever the vLP senses a ventricular event or paces the right ventricle.

The i2i messages that are sent between LPs may be relatively short messages that simply allow a first LP to inform a second LP of an event that was sensed by the first LP or caused (paced) by the first LP, and vice versa. Such i2i messages can be referred to herein as event marker i2i messages, or more succinctly as event i2i messages, or even more succinctly as event messages. The i2i messages that are sent between LPs, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection and correction code.

In certain embodiments, whenever an i2i message is sent by an LP (or other type of IMD, such as a S-ICD), the i2i message will include an extension indicator so that the receiving LP knows whether or not the i2i message it receives includes an extension portion. In such embodiments, even a relatively short event i2i message will include an extension indicator. The extension indicator (e.g., 2 bits) is used by the LP (or other IMD) sending the i2i message to indicate, to the LP receiving the i2i message, whether or not the i2i message is an extended i2i message. In certain embodiments, if the LP receiving an i2i message determines based on the extension indicator bits that the received i2i message is not an extended i2i message, then the LP receiving the i2i message can ignore any bits that follow the extension bits. In such a case, the LP receiving the i2i message only decodes the event marker. On the other hand, if the LP receiving an i2i message determines based on the extension indicator bits that the received i2i message is an extended i2i message, then the LP receiving the i2i message will also decode the bits that follow the extension bits, and determine based on a CRC code (or other type of error detection and correction code), whether or not the i2i message is a valid message. If the extended i2i message is a valid i2i message, then the LP receiving the extended i2i message will as appropriate modify its operation, update parameters, and/or the like, based on information included in the extended i2i message. In certain embodiments, event i2i messages that are not extended i2i messages do not include any error detection and correction code.

In an extended i2i message, the event marker bits and the extension indicator bits are located, respectively, in an event marker field and an extension indicator field of an i2i message packet. In certain embodiments, the extended portion (that follows the event marker bits and the extension indicator bits) includes message bits (in a message field) and rate indicator bits (in a rate indicator field), which are parts of the payload. The payload can alternatively, or additionally, include other types of fields, such as an acknowledgement field that is used in certain situations for one LP to acknowledge reception of an i2i message from another LP of certain (e.g., critical) types of message.

More generally, various different types of information may be included within the payload of an extended i2i message. For one example, the payload can include a pacing rate indicator that enables one LP to inform another LP of a pacing rate. For example, assume that an LP system provides rate responsive pacing, wherein a pacing rate is adjusted in dependence on a patient's physical activity as detected, e.g., using an accelerometer, temperature sensor, and/or other type of sensor of an LP. In such an LP system, the vLP may inform the aLP of the rate at which the patient's heart should be paced so that the aLP and vLP can perform synchronized pacing. To achieve this, the vLP can send a pacing rate indicator to the aLP in the payload of an extended i2i message. The pacing rate indicator can, e.g., be a value indicating a pacing rate value (e.g., 80 bpm), a code that the aLP that can look up (e.g., in a stored look up table) and corresponds to a pacing rate value, or a value that the aLP feeds into an equation to determine the pacing rate, but is not limited thereto. Alternatively, the pacing rate indicator can be beat-to-beat interval value (e.g., 0.75 seconds), a code that the aLP can look up and corresponds to a beat-to-beat interval value, or a value that the aLP feeds into an equation to determine the beat-to-beat interval, but is not limited thereto. Other variations are also possible and within the scope of the embodiments described herein.

As noted above, in the Background, the longevity of implantable medical devices (IMDs), such as LPs, is dependent on many factors, including the power consumed by such IMDs to perform i2i communication. Dual chamber LP systems may utilize i2i communication to provide for synchronous cardiac pacing. One possible i2i communication protocol that can be utilized by a dual chamber LP pacemaker system requires that an i2i event message is transmitted from one LP to another LP upon each paced and sensed event associated with the LP. For example, such an i2i communication protocol may require that an aLP transmits an atrial event message to the vLP each time the aLP detects an atrial sensed event and each time the aLP causes (or is about to cause) an atrial paced event. An atrial event message that informs the vLP of an atrial sensed event can be referred to herein more specifically as an atrial sensed event message. An atrial event message that informs the vLP of an atrial paced event can be referred to herein more specifically as an atrial paced event message. Accordingly, the general use of term atrial event message, as used herein, can refer to either an atrial sensed event message or an atrial paced event message. Where conductive communication is used to transmit such i2i messages, i.e., where i2i messages are conductive communication messages, the communication between LPs is relatively power efficient. Nevertheless, any opportunity to reduce the communication output burden of an LP will translate into a corresponding increase in the LP's overall longevity. Further, where an aLP has a smaller battery than a vLP, as may be the case in some dual chamber LP systems, it is even more important to reduce the communication output burden of the aLP. This is also the case because an output impedance for an aLP may be lower than that for a vLP.

For patients indicated for a dual-chamber pacemaker system due to AV Node block, their atrial-based sinus node generally functions well wherein it generates an appropriate intrinsic rhythm, but the subsequent propagation of that intrinsic signal from the atria to the ventricles through the AV node is blocked or substantially delayed. As such, a dual-chamber pacemaker system functions to bridge the dysfunctional AV node by sensing the intrinsic atrial events and then correspondingly pacing the ventricle after an appropriate AV delay. Where a dual-chamber LP system is implanted in such a patient, an aLP senses each intrinsic atrial event and may transmit an atrial event message to the vLP to notify (aka inform) the vLP of each atrial sensed event. Upon receipt of that atrial event message, the vLP may initiate an AV interval timer and then pace the ventricle when that timer expires. The vLP may also transmit a ventricular event message back to the aLP notifying the aLP of the ventricular paced event, or when appropriate, of a ventricular sensed event. In such a dual-chamber LP system, the aLP may also transmit an atrial event message to the vLP each time the aLP causes (or is about to cause) a paced atrial event, and the vLP may respond accordingly to receiving such a message.

Certain embodiments of the present technology described herein provide for an updated i2i communication protocol between LPs, wherein the aLP selectively withholds the transmission of an atrial event message for an atrial event (e.g., an atrial sensed event) when the underlying atrial rate (e.g., intrinsic atrial rate) is relatively consistent/steady across two or more consecutive cardiac cycles, where the criteria that defines "relatively consistent" can be specified by a user (e.g., clinician and/or physician). More specifically, in accordance with certain embodiments of the present technology, the aLP selectively abstains from transmitting atrial event messages to the vLP, and thereby selectively abstains from informing the vLP of certain atrial events, which has the effect of reducing power consumption (associated with the aLP transmitting atrial event messages to the vLP) and thereby increasing the expected longevity of the aLP.

Consider a patient with competent atrial intrinsic activity but a dysfunctional AV node. In this scenario, the patient requires a dual-chamber pacemaker system that can provide ventricular pacing which tracks atrial activity with an appropriate AV delay to enable sufficient filling of the ventricular chambers prior to its paced-induced contraction of the ventricles. Dual-chamber pacemaker systems generally enable a clinician to program a specific value for the sensed AV delay (aka the PV delay) that is appropriate for that patient. In reality, however, there may actually be a range of AV delay values that are sufficiently therapeutic for the patient without a clinically meaningful loss of cardiac function. Certain embodiments of the present technology leverage this concept to reduce the frequency with which (i.e., how often) an aLP needs to transmit atrial event messages to a vLP in a dual chamber LP system.

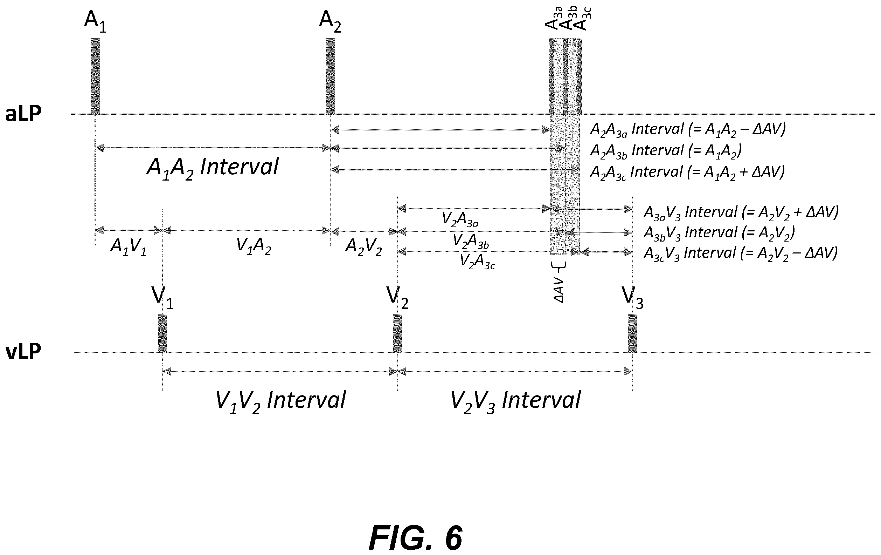

With reference to the timing diagram of FIG. 6, assume that the aLP detects a series of intrinsic atrial events (labeled $A_1$, $A_2$, $A_3$, etc.) and that the vLP tracks those atrial events (via receipt of associated i2i messages sent by the aLP to the vLP) and paces the right ventricular chamber after defined AV intervals ($A_1V_1$, $A_2V_2$, $A_3V_3$, etc.). In such a dual chamber LP system, the vLP can be configured such that the vLP has a backup ventricular-to-ventricular interval timer (VV interval timer) that is used when the vLP fails to receive a valid atrial event message from the aLP during a cardiac cycle. More specifically, if the vLP does not receive a valid atrial event message from the aLP during a cardiac cycle, the vLP is unable to start an AV interval timer, and thus will instead utilize its backup VV interval timer that it started upon delivery of a previous ventricular pacing pulse (or an intrinsic ventricular event) to determine the timing of the next ventricular pace pulse. In FIG. 6, each VA interval is an interval between a paced or intrinsic ventricular event and an immediately succeeding (aka following or subsequent) paced or intrinsic atrial event.

The AV interval referred to above could be a programmed value of a sensed AV delay (AVD), or it could be a modified interval, such as implemented via a rate-responsive AV delay algorithm, etc. Certain embodiments of the present technology provide an ability to define a range of acceptable AV delay values instead of a singular acceptable value of AV delay. This range can be defined as an absolute range (e.g., $[AVD_{min}, AVD_{max}]$), as absolute deltas from a target AV delay (e.g., $[AVD_{target} - AVD_{\Delta}, AVD_{target} + AVD_{\Delta}]$, or $[AVD_{target} - AVD_{\Delta 1}, AVD_{target} + AVD_{\Delta 2}]$, etc), as a relative percentage from a target AV delay (e.g., $[AVD_{target} * (1 - AVD_{\Delta\%}), AVD_{target} * (1 + AVD_{\Delta\%})]$, etc), or any other applicable means to define that range.

When a patient's cardiac rate (aka heart rate) is steady-state, the following relationships are assumed to be true:

$$A_nA_{n+1} \text{ interval} = A_nV_n \text{ interval} + V_nA_{n+1} \text{ interval},$$

$$V_nV_{n+1} \text{ interval} = V_nA_{n+1} \text{ interval} + A_{n+1}V_{n+1} \text{ interval},$$

and

$$A_{n+1}V_{n+1} \text{ interval} = A_nV_n \text{ interval}.$$

Certain embodiments of the present technology described herein take advantage of the realization that a certain amount of variation in the AA interval can be tolerated while enabling a dual chamber LP system to continue to utilize the steady-state VV interval and still maintain a functional AV interval within the defined range of permitted AV delays. Because of the linearity of the relationships above (and from inspection of FIG. 6), the AA interval can vary by the same amount of the permitted range of AV delays. Mathematically, if this permitted range is defined as $A_{n+1}V_{n+1} = A_nV_n \pm \Delta AV$, then:

$$V_nV_{n+1} = V_nA_{n+1} + A_{n+1}V_{n+1},$$

$$V_nV_{n+1} = V_nA_{n+1} + (A_nV_n \pm \Delta AV),$$

$$V_nV_{n+1} = (V_nA_{n+1} + A_nV_n) \pm \Delta AV,$$

$$V_nV_{n+1} = (A_nA_{n+1}) \pm \Delta AV,$$

and

$$A_nA_{n+1} = V_nV_{n+1} \mp \Delta AV.$$

Therefore, in accordance with certain embodiments, when a present AA interval matches the immediately preceding AA interval *to* within $\pm \Delta AV$, then the aLP withholds the i2i atrial event message that is usually used to notify the vLP of that atrial sensed event, and instead the vLP relies on the previously initiated VV interval timer to drive the timing of a ventricular paced event that will still provide effective AV tracking to within the defined $\pm \Delta AV$ tolerance.

As an example, consider a patient for whom the sensed AV delay (aka the PV delay) is programmed to be 200 msec. Also consider that a clinician has determined that any AV interval within the range of 150 msec to 250 msec (i.e., 200 $\pm$ 50 msec such that $\Delta AV = 50$ msec) would be acceptable. That means that if the patient's intrinsic atrial rate is approximately 60 bpm (which correspond to an VV interval of 1000 msec), then the aLP could withhold (i.e., abstain from) transmitting an i2i atrial event message to the vLP if the intrinsic atrial interval remains within 1000 $\pm$ 50 msec, in which case the subsequent AV interval will still meet the targeted 200 $\pm$ 50 msec window.

To extend this concept over multiple consecutive cardiac cycles, certain embodiments of the present technology ensure that each cycle continues to meet this AV tolerance criteria, thereby preventing a net accumulation of deltas across those consecutive cycles from exceeding the originally-defined tolerance window.

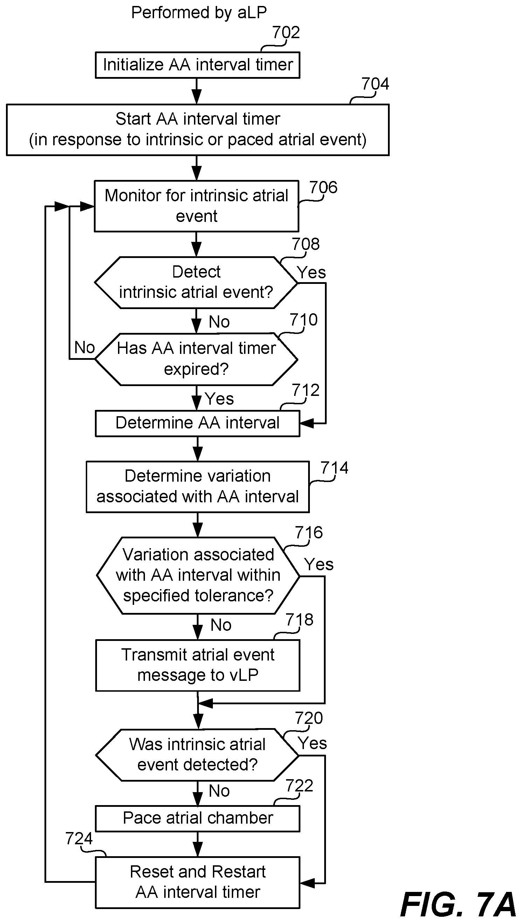

Certain embodiments of the present technology are related to methods, described below with reference to the flow diagrams of FIGS. 7A-7D and 8-10, which are for use by an implantable dual chamber LP system including an aLP implanted in or on an atrial chamber (e.g., the right atrial chamber, aka the right atrium) and a vLP implanted in or on a ventricular chamber (e.g., the right ventricular chamber, aka the right ventricle). Embodiments of the present technology are also directed to LPs and dual chamber LP systems that are configured to perform such methods, or more specifically, the features of such methods. Certain such embodiments are used by an aLP to selectively abstain from transmitting atrial event messages to a vLP, to thereby conserve power of the aLP, which in turn increases the longevity of the aLP. Other embodiments are utilized by a vLP such that the vLP can appropriately pace a ventricle when the vLP does not receive an atrial event message from an aLP because the aLP had purposely abstained from transmitting the atrial event message to conserve its power.

FIGS. 7A-7D will initially be used to summarize certain methods that are performed by an aLP, such as the aLP 102b described above, but not limited thereto. Referring to FIG. 7A, step 702 involves the aLP initializing its AA interval timer to an initial AA interval value. The initial AA interval value can be a base AA interval value that is programmed or set by default. Alternatively the initial AA interval value can be a rate responsive value that depends on an activity level of the patient, which can be determined using a temperature sensor and/or an accelerometer, but not limited thereto. The AA interval timer (e.g., 115) can be configured to count-up, or count-down, for the AA interval value once initiated, at which point the sleep timer expires. Where the AA interval timer is a count-down timer, initializing (or resetting) the AA interval timer means setting it to the AA interval value, and expiration of the AA interval timer occurs when the timer reaches zero. Where the AA interval timer is a count-up timer, initializing (or resetting) the AA interval timer means setting it to zero, and expiration of the AA interval timer occurs when the timer reaches the AA interval value. Other timers (e.g., 115) mentioned herein can similarly be either configured as count-up or count-down timers. Step 704 involves starting the AA interval timer in response to an atrial sensed or paced event. Thereafter, the aLP monitors for an intrinsic atrial event 706, using any known or future developed technique. This can involve, for example, the aLP sensing an EGM using its electrodes (e.g., 108) and sense amplifier (e.g., 132) and monitoring for a P-wave in the EGM, wherein a P-wave corresponds to an intrinsic atrial depolarization (activation), as is known in the art. At step 708 there is a determination of whether or not an intrinsic atrial event has been detected. If the answer to the determination at step 708 is No, then flow goes to step 710 where there is a determination of whether or not the AA interval timer has expired. If the answer to the determination at step 710 is No, then flow returns to step 706, and the aLP continues to monitor for an atrial event. If the answer to the determination at step 708 is Yes, then flow goes to step 712, at which point the AA interval associated with the detected atrial event is determined, wherein the AA interval corresponds to a duration between the atrial event just detected and an immediately preceding atrial event. Similarly, if the answer to the determination at step 710 is Yes, then flow goes to step 712, at which point the AA interval associated with the detected atrial event is determined. As shown in FIG. 7A, an AA interval may also be determined at an instance of step 712 in response to an intrinsic atrial event being detected, and more specifically, when the answer to the determination at step 708 is Yes. After the AA interval is determined at step 712, flow goes to step 714.

At step 714 a variation associated with the AA interval is determined, and then at step 716 there is a determination of whether or not the variation associated with the AA interval is within a specified tolerance. While step 714 and 716 are shown as two separate steps in FIG. 7A, as well as in other flow diagrams, such steps can equivalently be implemented as a single step that involves determining whether or not a variation associated with the AA interval is within a specified tolerance. If the answer to the determination at step 716 is No, i.e., if the AA interval is not within the specified tolerance, then flow goes to 718, wherein at step 718 the aLP transmits an atrial event message to the vLP to thereby inform the vLP of the atrial event that was sensed (i.e., of an intrinsic atrial event), or of an atrial paced event that is about to occur (responsive to the AA interval timer expiring before an intrinsic atrial event was detected). In accordance with certain embodiments, the atrial event message that the aLP transmits to the vLP at step 718 can include an updated ventricular target interval for the vLP to start using.

Still referring to FIG. 7A, at step 720 there is a determination of whether or not an intrinsic atrial event was sensed, and more specifically was detected at step 708. If the answer to step 720 is No, then flow goes to step 722, wherein at step 722 the aLP delivers a pacing pulse to the atrial chamber (in or on which the aLP is implanted). The AA interval timer is then reset (e.g., to the same value to which the AA interval timer was initialized at step 702) and restarted at step 724. If the answer to step 720 is Yes, then there is no need to pace that atrial chamber and flow skips step 722 and goes to step 724. While step 724 is shown as a single step, it could alternatively be separated into two steps, one what involves resetting the AA interval timer, and another that involves restarting the AA interval timer. Such resetting of the AA interval timer can alternatively occur as soon as an intrinsic atrial event is detected (e.g., as part of or following step 708), or as soon the AA interval timer has expired (as determined at step 710), or when the atrial chamber is paced by the aLP. Other variations are also possible and within the scope of the embodiments described herein.

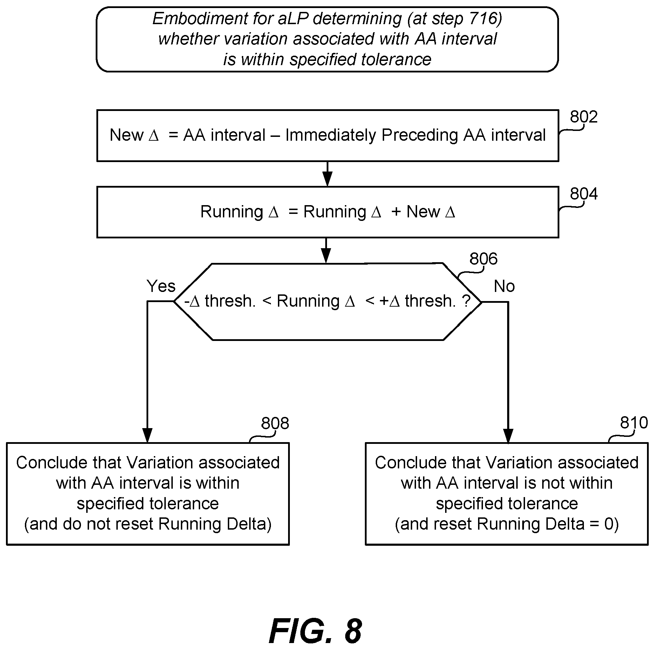

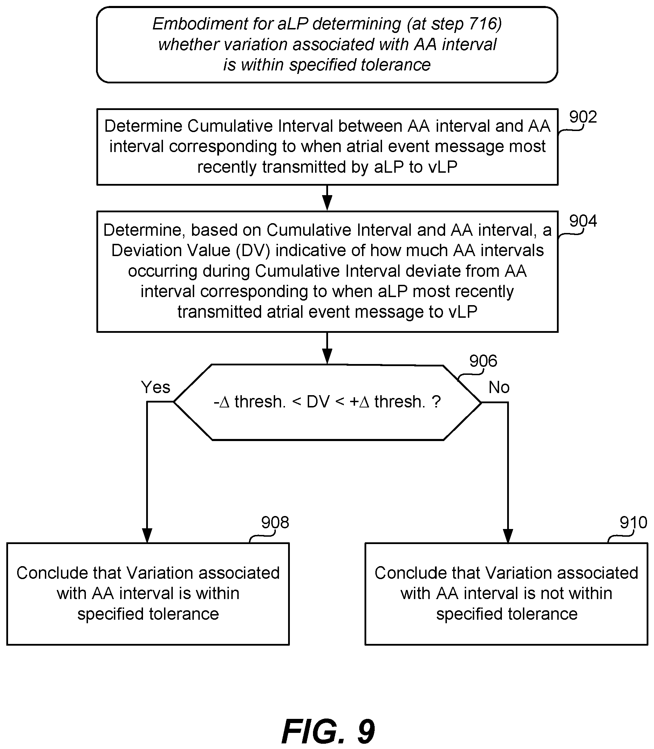

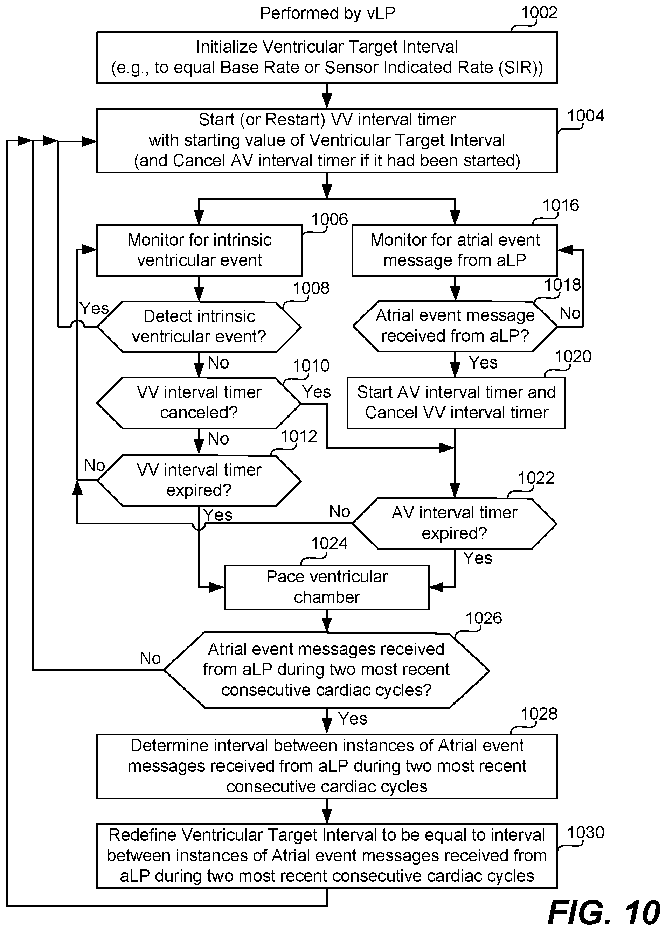

Returning to step 716, if the answer to the determination at step 716 is Yes, i.e., if the AA interval (determined at the most recent instance of step 712) is within the specified tolerance, then flow skips step 718. In this manner, the aLP abstains from transmitting the atrial event message to the vLP (and thereby abstains from informing the vLP of the atrial event) when that AA interval is within the specified tolerance, thereby conserving the aLP's power. As noted above, and explained in more detail below with reference to FIG. 10, when the aLP abstains from transmitting the atrial event message to the vLP (and thereby abstains from informing the vLP of the atrial event), the vLP relies on its VV interval timer to know whether and when it should pace the ventricular chamber (in or on which the vLP is implanted). Additional details as to how the aLP can determine whether or not the AA interval is within the specified tolerance are described below, e.g., with reference to FIGS. 8 and 9.

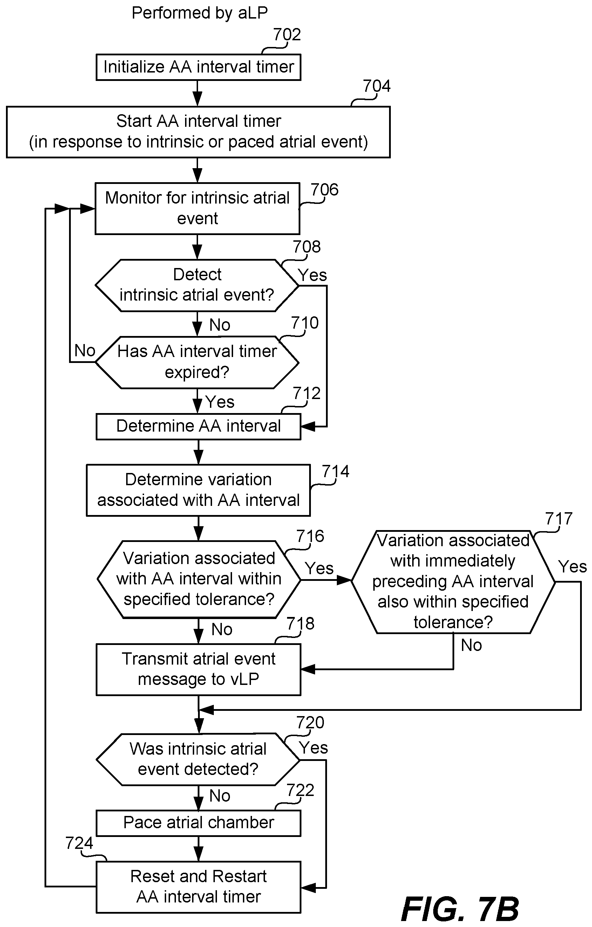

In the embodiments summarized above with reference to FIG. 7A, the aLP abstains from transmitting an atrial event message to the vLP when the aLP determines that a variation associated with the AA interval (corresponding to an intrinsic atrial event just detected by the aLP, or a paced atrial event about to be caused by the aLP) is within the specified tolerance. In other embodiments, summarized below with reference to FIG. 7B, the aLP only abstains from transmitting an atrial event message to the vLP when the aLP determines that a variation associated with the AA interval (corresponding to an intrinsic atrial event just detected by the aLP, or a paced atrial event about to be caused by the aLP) is within the specified tolerance, and a variation associated with an immediately preceding AA interval (corresponding to an immediately preceding intrinsic atrial event detected by the aLP, or an immediately preceding paced atrial event caused by the aLP) was also within the specified tolerance. Such embodiments, summarized with reference to FIG. 7B, ensure that at least two consecutive atrial event messages are transmitted from the aLP to the vLP, following an AA interval (corresponding to an intrinsic atrial event just detected by the aLP, or a paced atrial event about to be caused by the aLP) whose associated variance is not within the specified tolerance. A benefit of such embodiments is that they enable the vLP to accurately update (e.g., redefine) its ventricular target interval when appropriate.

Referring now to FIG. 7B, the steps therein that are the same as in FIG. 7A are labeled the same and need not be described again, because they can be understood from the above description of FIG. 7A. A comparison between FIGS. 7B and 7A shows that the only difference between them is the addition of step 717 in FIG. 7B. More specifically, in FIG. 7B, when the answer to the determination at step 716 is Yes (i.e., when the variation associated with the AA interval is within the specified tolerance), flow goes to step 717. At step 717 there is a determination of whether or not the AA interval associated with an immediately preceding atrial event was also within the specified tolerance. If the answer to the determination at step 717 is No, then flow goes to step 718, and the aLP transmit an atrial event message to the vLP, to thereby inform the vLP of an intrinsic atrial event just detected by the aLP (at the most recent instance of step 708), or a paced atrial event about to be caused by the aLP (at an instance of step 722). If the answer to the determination at step 717 is Yes, then step 718 is skipped and the aLP thereby abstains from transmitting an atrial event message to the vLP, and thereby abstains from informing the vLP of an intrinsic atrial event just detected by the aLP (at the most recent instance of step 708), or a paced atrial event about to be caused by the aLP (at an instance of step 722). As can be appreciated from FIG. 7B, and the description thereof, in such embodiments the aLP only abstains from transmitting an atrial event message to the vLP when the aLP determines that a variation associated with the AA interval (corresponding to an intrinsic atrial event just detected by the aLP, or a paced atrial event about to be caused by the aLP) is within the specified tolerance, and a variation associated with an immediately preceding AA interval (corresponding to an immediately preceding intrinsic atrial event detected by the aLP, or an immediately preceding paced atrial event caused by the aLP) was also within the specified tolerance.

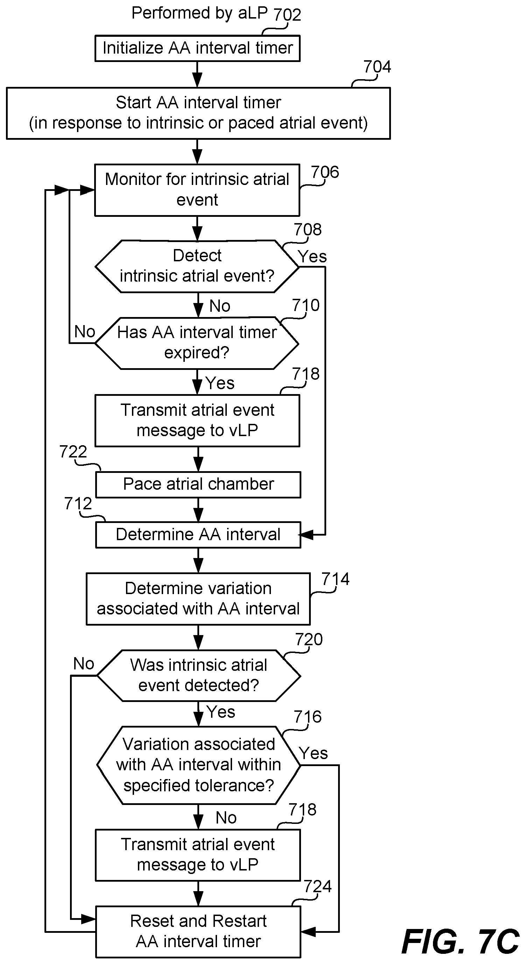

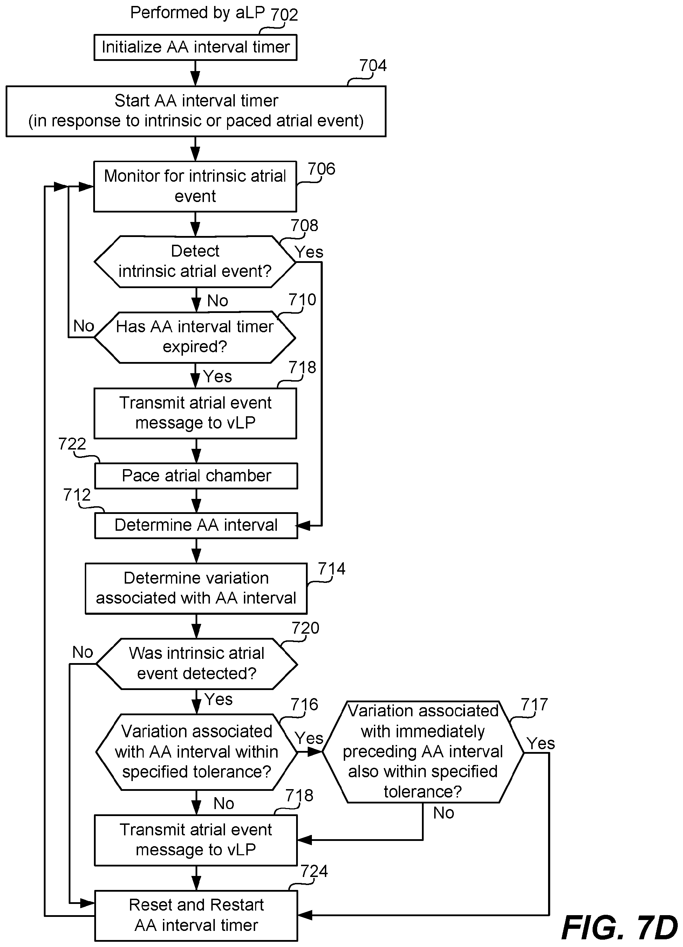

In the embodiments summarized above with reference to FIGS. 7A and FIG. 7B, the aLP selectively abstains from transmitting an atrial event message to the vLP, based on whether or not the AA interval corresponding to the atrial event is within a specified tolerance, regardless of whether the atrial event (for which the atrial event message is not sent) is an intrinsic atrial event or a paced atrial event. In other embodiments, summarized below with reference to FIGS. 7C and 7D, the aLP only selectively abstains from transmitting an atrial event message to the vLP, based on whether or not the AA interval corresponding to the atrial event is within a specified tolerance, where the atrial event (for which the atrial event message is not transmitted) is an intrinsic atrial event. In other words, in the embodiments summarized with reference to FIGS. 7C and 7D, the aLP will always transmit an atrial paced event type of atrial event message, regardless of whether or not the AA interval corresponding to the atrial event is within the specified tolerance. That is, in such embodiments, the aLP only selectively abstains from transmitting atrial event messages to the vLP where the atrial event messages are intended to inform the vLP of atrial sensed events, but not where the atrial event messages are intended to inform the vLP of atrial paced events. A benefit of the embodiments summarized below with reference to FIGS. 7C and 7D, is that such embodiments enable the vLP to initiate one or more blanking periods, and/or the like, to reduce and preferably prevent any crosstalk detection by the vLP, and more specifically, prevents the vLP from mistakenly detecting and interpreting atrial pacing stimulation as a ventricular sensed event.

Referring now to FIG. 7C, the steps therein that are the same as in FIG. 7A are labeled the same and need not be described again, because they can be understood from the above description of FIG. 7A. A comparison between FIGS. 7C and 7A shows that steps 702, 704, 706, 708, and 710 are the same, and are performed in the same order in FIG. 7C as in FIG. 7A. However, in FIG. 7C step 718 occurs whenever the answer to the determination at step 710 is Yes (i.e., whenever there is a determination that the AA interval timer has expired), because in the embodiments summarized with reference to FIG. 7C an atrial paced event type of atrial event message is always transmitted from the aLP to the vLP. Following step 718, the aLP paces the atrial chamber at step 722, and the AA interval is determined at step 712. It would also be possible to reverse the order of steps 722 and 712, such that step 712 occurs prior to step 722, since the aLP knows when it is going to pace the atrial chamber. Still referring to FIG. 7C, at step 714 there is a determination of the variation associated with AA interval determined at the most recent instance of step 712. Following step 714 there is a determination at step 720 of whether or not an intrinsic atrial event was detected at step 708. If the answer to the determination at step 720 is No, that means that the atrial event was a paced atrial event for which an atrial event message was already transmitted by the aLP to the vLP at step 718, and flow goes to step 724. If the answer to the determination at step 720 is Yes, then step 716 is performed for the purpose of determining whether or not the aLP should abstain from sending an atrial event message to the vLP and thereby abstain from informing the vLP of the atrial sensed event.

At step 716 there is a determination of whether or not the variation associated with the AA interval is within a specified tolerance. If the answer to the determination at step 716 is No, i.e., if the AA interval is not within the specified tolerance, then flow goes to 718, wherein at step 718 the aLP transmits an atrial event message to the vLP to thereby inform the vLP of the atrial event that was sensed (i.e., of an intrinsic atrial event). If the answer to the determination at step 716 is Yes, i.e., if the AA interval (determined at the most recent instance of step 712) is within the specified tolerance, then flow skips step 718. In this manner, the aLP abstains from transmitting the atrial event message to the vLP (and thereby abstains from informing the vLP of the atrial event) when that AA interval associated with the intrinsic atrial event is within the specified tolerance, thereby conserving the aLP's power. As noted above, and explained in more detail below with reference to FIG. 10, when the aLP abstains from transmitting the atrial event message to the vLP (and thereby abstains from informing the vLP of the atrial event), the vLP relies on its VV interval timer to know whether and when it should pace the ventricular chamber (in or on which the vLP is implanted). As noted above in the discussion of FIG. 7A, additional details as to how the aLP can determine whether or not the AA interval is within the specified tolerance are described below, e.g., with reference to FIGS. 8 and 9.

In the embodiments summarized above with reference to FIG. 7C, the aLP abstains from transmitting an atrial event message to the vLP when the aLP determines that a variation associated with the AA interval (corresponding to an intrinsic atrial event just detected by the aLP) is within the specified tolerance. In other embodiments, summarized below with reference to FIG. 7D, the aLP only abstains from transmitting an atrial event message to the vLP when the aLP determines that a variation associated with the AA interval (corresponding to an intrinsic atrial event just detected by the aLP) is within the specified tolerance, and a variation associated with an immediately preceding AA interval (corresponding to an immediately preceding intrinsic atrial event detected by the aLP, or an immediately preceding paced atrial event caused by the aLP) was also within the specified tolerance. Such embodiments, summarized with reference to FIG. 7D, ensure that at least two consecutive atrial event messages are transmitted from the aLP to the vLP, following an AA interval (corresponding to an intrinsic atrial event just detected by the aLP) whose associated variance is not within the specified tolerance. A benefit of such embodiments is that they enable the vLP to accurately update (e.g., redefine) its ventricular target interval when appropriate.

Referring now to FIG. 7D, the steps therein that are the same as in FIG. 7C are labeled the same and need not be described again, because they can be understood from the above description of FIG. 7C. A comparison between FIGS. 7D and 7C shows that the only difference between them is the addition of step 717 in FIG. 7D. More specifically, in FIG. 7D, when the answer to the determination at step 716 is Yes (i.e., when the variation associated with the AA interval is within the specified tolerance), flow goes to step 717. At step 717 there is a determination of whether or not the AA interval associated with an immediately preceding atrial event was also within the specified tolerance. If the answer to the determination at step 717 is No, then flow goes to step 718, and the aLP transmit an atrial event message to the vLP, to thereby inform the vLP of an intrinsic atrial event just detected by the aLP (at the most recent instance of step 708). If the answer to the determination at step 717 is Yes, then step 718 is skipped and the aLP thereby abstains from transmitting an atrial event message to the vLP, and thereby abstains from informing the vLP of an intrinsic atrial event just detected by the aLP (at the most recent instance of step 708). As can be appreciated from FIG. 7D, and the description thereof, in such embodiments the aLP only abstains from transmitting an atrial event message to the vLP when the aLP determines that a variation associated with the AA interval (corresponding to an intrinsic atrial event just detected by the aLP) is within the specified tolerance, and a variation associated with an immediately preceding AA interval (corresponding to an immediately preceding intrinsic atrial event detected by the aLP, or an immediately preceding paced atrial event caused by the aLP was also within the specified tolerance.

In the embodiments summarized above with reference to FIGS. 7A through 7D, an atrial event message that is a paced atrial event message, when transmitted from the aLP to the vLP, is transmitted by the aLP to the vLP just prior to (e.g., approximately 1 to 5 msec before) the aLP delivering pacing stimulation to the atrium (in or on which the aLP is implanted). This is because the transmitting at step 718 (when performed) is shown as occurring prior to the pacing of the atrial chamber at step 722 (when performed). As noted above, a benefit of the aLP transmitting an atrial paced event type of atrial event message to the vLP prior to the aLP performing atrial pacing is that the atrial event message (transmitted prior to atrial pacing) informs the vLP of the impending pacing stimulation pulse, thereby enabling the vLP to initiate one or more blanking periods, and/or the like, to reduce and preferably prevent any crosstalk detection by the vLP, and more specifically, prevents the vLP from mistakenly detecting and interpreting atrial pacing stimulation as a ventricular sensed event. Nevertheless, in alternative embodiments the aLP transmits an atrial paced event type of atrial event message to the vLP just after (e.g., approximately 1 to 5 msec after) the aLP delivers atrial pacing stimulation to the atrial chamber (in or on which the aLP is implanted), in which case the vLP can utilize cross talk mitigation techniques and/or other types of techniques to mitigate the probability of the vLP mistakenly interpreting the atrial pacing stimulation as a ventricular sensed event. For example, the vLP may use one or more morphology templates stored in memory of the vLP to determines signal components within a signal sensed by the vLP correspond to an atrial paced event, to prevent the vLP from mistakenly interpreting atrial pacing stimulation as a ventricular sensed event. For more specific examples, techniques described in commonly assigned U.S. Patent Nos. 10,182,765 and 10,993,674 may be used by the vLP to prevent the vLP from mistakenly interpreting atrial pacing stimulation as a ventricular sensed event. Other variations are also possible and within the scope of the embodiments described herein. In such an alternative embodiment, the aLP may pace the atrial chamber as soon as the AA interval timer expires, e.g., immediately following there being a Yes determination at step 710, or at any other time prior to step 718, in which case step 720 can be eliminated. Other variations are also possible, and within the scope of the embodiments described herein.

In FIGS. 7A-7D, at steps 714 and 716, or at step 714, 716, and 717, the aLP determines, based on results of determining whether or not a variation associated with an AA interval is within a specified tolerance, whether or not an atrial event message should be transmitted to the vLP to inform the vLP of the atrial event. Thereafter, the aLP, in response to determining that the atrial event message should not be transmitted to the vLP, abstains from transmitting the atrial event message to the vLP and thereby abstains from informing the vLP of the atrial event. Alternatively, when the aLP determines that the atrial event message should be transmitted to the vLP, the aLP transmits the atrial event message to the vLP and thereby informs the vLP of the atrial event. One of ordinary skill in the art reading this disclosure will understand that the aLP may perform alternative and/or additional steps to determine whether or not a variation associated with an AA interval is within a specified tolerance, and/or to determine whether or not an atrial event message should be transmitted to the vLP to inform the vLP of the atrial event, while being within the scope of the embodiments described herein.

One way for the aLP to determine whether or not the variation associated with the AA interval is within a specified tolerance, at step 716, is as follows. The aLP can determine a delta (i.e., difference) between the AA interval and an AA interval corresponding to an immediately preceding atrial event, and the aLP can compare the determined delta (i.e., difference) to the specified threshold. The aLP can then determine that the variation associated with the AA interval is within the specified tolerance when the delta is within the specified tolerance, or the aLP can determine that the variation associated with the AA interval is not within the specified tolerance when the determined delta is not within the specified tolerance. More specifically, the aLP can determine whether or not the determined delta is within a range specified by a $-\Delta$ threshold and a $+\Delta$ threshold, i.e., the aLP can determine whether $-\Delta$ threshold < determined delta < $+\Delta$ threshold. In such embodiments, the absolute value of the $-\Delta$ threshold can be the same as the absolute value of the $+\Delta$ threshold, but that need not be the case, depending upon the specific implementation. In certain embodiments, the value of $-\Delta$ threshold and the value of the $+\Delta$ threshold can be fixed values, e.g., the $-\Delta$ threshold can be -50 msec and the $+\Delta$ threshold can be +50 msec. In other embodiments, the value of $-\Delta$ threshold and the value of the $+\Delta$ threshold can be variable, e.g., can depend in the patient's atrial rate, and more specifically, a most recently determined AA interval. For example, the $-\Delta$ threshold can be equal to the product of -0.05 * AA interval, and the $+\Delta$ threshold can be equal to the product of - 0.05 * AA interval. In such an embodiment, when the AA interval is 1000 msec, then $-\Delta$ threshold is set to -0.05 * 1000 msec = - 50 msec, and the $+\Delta$ threshold is set to 0.05 * 1000 msec = 50 msec. However, if the patient's atrial rate increases, thereby reducing the AA interval, e.g., to 900 msec, then $-\Delta$ threshold is set to -0.05 * 900 msec = - 45 msec, and the $+\Delta$ threshold is set to 0.05 * 900 msec = 45 msec. Other variations are also possible and within the scope of the embodiments described herein. A potential detriment of such embodiments is that it is possible that they may not enable the vLP to appropriately adjust its ventricular target interval if AA intervals detected by the aLP slowly drift over a number of consecutive cardiac cycles, which may result in ventricular pacing performed by the vLP not tracking atrial activity as closely as desired. To avoid such a potential detriment, the aLP may be configured such that the aLP only abstains from transmitting an atrial event message to the vLP where a net accumulation of deltas across consecutive cardiac cycles remains with the specified tolerance. There are various ways that the aLP can determine whether or not a net accumulation of deltas across consecutive cardiac cycles remains with the specified tolerance, some of which are described below with reference to FIGS. 8 and 9.

Referring to FIG. 8, it is presumed that a running $\Delta$ (initially referred to in step 804) is initialized to zero by the aLP when the aLP starts to operate, or at some other point(s) in time, e.g., at instances of step 810 described below. At step 802 the aLP calculates a new delta (new $\Delta$) as being equal to the AA interval (just determined by the aLP) minus the immediately preceding AA interval (i.e., the AA interval associated with the immediately preceding atrial event). At step 804 the aLP calculates a running delta (running $\Delta$) as being equal to a sum of the most recently calculated (or reset) running $\Delta$ plus the new $\Delta$ (just calculated at the most recent instance of step 802). At step 806 the aLP determines whether or not the running $\Delta$ is within the specified tolerance, and more specifically determines whether $-\Delta$ threshold < running delta < $+\Delta$ threshold, or alternatively whether $-\Delta$ threshold $\leq$ running delta $\leq$ $+\Delta$ threshold. In certain embodiments, the value of $-\Delta$ threshold and the value of the $+\Delta$ threshold can be fixed values, e.g., the $-\Delta$ threshold can be -50 msec and the $+\Delta$ threshold can be +50 msec. In other embodiments, the value of $-\Delta$ threshold and the value of the $+\Delta$ threshold can be variable, e.g., can depend in the patient's atrial rate, and more specifically, a most recently determined AA interval. Example details of how the value of $-\Delta$ threshold and the value of the $+\Delta$ threshold can be variable were described above, and thus need not be repeated. If the answer to the determination at step 806 is Yes, then flow goes to step 808, and the aLP concludes that the variation associated with the AA interval is within the specified tolerance, and the aLP does not reset the running $\Delta$. If the answer to the determination at step 806 is No, then flow goes to step 810, and the aLP concludes that the variation associated with the AA interval is not within the specified tolerance, and the aLP resets the running $\Delta$.

Alternative embodiments for the aLP determining whether or not a net accumulation of deltas across consecutive cardiac cycles remains with the specified tolerance are now described with reference to FIG. 9. Referring to FIG. 9, step 902 involves determining a cumulative interval between the AA interval (most recently determined) and an AA interval corresponding to when the aLP most recently transmitted the atrial event message to the vLP. Assume that the atrial event corresponding to when the aLP most recently transmitted the atrial event message to the vLP can be referred to as $A_0$, a first atrial event following when the aLP most recently transmitted the atrial event message to the vLP can be referred to as $A_1$, a second atrial event following when the aLP most recently transmitted the atrial event message to the vLP can be referred to as $A_2$, ... and more generally that an nth atrial event following when the aLP most recently transmitted the atrial event message to the vLP can be referred to as $A_n$. Also assume that the atrial event occurring immediately preceding when the aLP most recently transmitted the atrial event message to the vLP can be referred to as $A_{-1}$. Using this naming convention, the AA interval corresponding to when the aLP most recently transmitted the atrial event message to the vLP can be represented as $A_{-1}A_0$, and the AA interval (most recently determined) for the nth atrial event can be represented as $A_{n-1}A_n$, and the cumulative interval between the AA interval (most recently determined) for the nth atrial event (following when the aLP most recently transmitted the atrial event message to the vLP) and when the aLP most recently transmitted the atrial event message to the vLP can be represented as $A_0A_n$.

Still referring to FIG. 9, step 904 involves determining, based on the cumulative interval (i.e., $A_0A_n$) and the AA interval (i.e., $A_{n-1}A_n$) (most recently determined) for the nth atrial event that occurred since the aLP most recently transmitted the atrial event message to the vLP, a deviation value (DV) indicative of how much AA intervals occurring during the cumulative interval (i.e., during $A_0A_n$) deviate from the AA interval (i.e., $A_{-1}A_0$) corresponding to when the aLP most recently transmitted the atrial event message to the vLP. In accordance with certain embodiments, the deviation value (DV) can be calculated using the equation $DV = A_0A_n - n*A_{-1}A_0$. In such embodiments, the deviation value (DV) is the variation associated with the AA interval.

Still referring to FIG. 9, at step 906 the aLP determines whether or not the deviation value (DV) is within the specified tolerance, and more specifically determines whether $-\Delta$ threshold $< DV < +\Delta$ threshold, or alternatively whether $-\Delta$ threshold $\leq DV \leq +\Delta$ threshold. In certain embodiments, the value of $-\Delta$ threshold and the value of the $+\Delta$ threshold can be fixed values, e.g., the $-\Delta$ threshold can be -50 msec and the $+\Delta$ threshold can be +50 msec. In other embodiments, the value of $-\Delta$ threshold and the value of the $+\Delta$ threshold can be variable, e.g., can depend in the patient's atrial rate, and more specifically, a most recently determined AA interval. Example details of how the value of $-\Delta$ threshold and the value of the $+\Delta$ threshold can be variable were described above, and thus need not be repeated. If the answer to the determination at step 906 is Yes, then flow goes to step 908, and the aLP concludes that the variation associated with the AA interval is within the specified tolerance. If the answer to the determination at step 906 is No, then flow goes to step 910, and the aLP concludes that the variation associated with the AA interval is not within the specified tolerance.

One of ordinary skill in the art reading the above description will appreciate that there are many other ways that an aLP can determine whether or not an AA interval corresponding to an atrial event is within a specified tolerance for the purpose of determining whether or not the aLP should abstain from transmitting an atrial event message to a vLP, with such other ways also being within the scope of the embodiments described herein.

FIG. 10 will now be used to summarize how a vLP (e.g., 102b) of a dual chamber leadless pacemaker system can utilize its VV interval timer to pace the ventricular chamber (in or on which the vLP is implanted) when the aLP (e.g., 102a) abstains from sending an atrial event message to the vLP. FIG. 10 is also used to summarize how the vLP may update its ventricular target interval when appropriate, wherein the ventricular target interval is the starting value used by the VV interval timer of the vLP.

Referring to FIG. 10, step 1002 involves the vLP initializing its ventricular target rate to equal a base rate, or a sensor indicated rate, wherein the sensor indicated rate may depend on a patient's activity level as detected, e.g., using a temperature sensor and/or an accelerometer, but not limited thereto. Step 1004 involves the vLP starting (or restarting) its VV interval timer with a starting value of the ventricular target rate, and canceling the AV interval timer if it had been started. The vLP then simultaneously monitors for an intrinsic ventricular event at step 1006 and for an atrial event message from the aLP at step 1016. In certain embodiments, step 1006 and/or step 1016 may not occur during a blanking period that may be initiated by the vLP for various reasons.

First referring to the left branch of the flow diagram, the vLP can monitor for an intrinsic ventricular event at step 1006 using any known or future developed technique. This can involve, for example, the vLP sensing an EGM using its electrodes (e.g., 108) and sense amplifier (e.g., 132) and monitoring for an R-wave in the EGM, wherein an R-wave corresponds to an intrinsic ventricular depolarization (activation), as in known in the art. At step 1008 there is a determination of whether or not an intrinsic ventricular event has been detected. If the answer to the determination at step 1008 is No, then flow goes to step 1010 where there is a determination of whether or not the VV interval timer has been cancelled (e.g., at an instance of step 1020). If the answer to the determination at step 1010 is No, the flow goes to step 1012 and there is a determination of whether or not the VV interval timer has expired. If the answer to the determination at step 1010 is Yes, then flow goes to step 1022, which is discussed below. If the answer to the determination at step 1012 is Yes, then flow goes to step 1024 and the vLP paces the ventricular chamber by delivering one or more pacing stimulation pulses to the ventricular chamber (in or on which the vLP is implanted). Following the vLP pacing the ventricular chamber at step 1024, flow goes to step 1026, which is discussed below.

Returning to step 1008, if there is a determination at step 1008 that the vLP detected an intrinsic atrial event, i.e., if the answer to the determination at step 1008 is Yes, then flow returns to step 1004, at which time the VV interval timer is restarted using a starting value of the most recently determined ventricular target value, and the AV interval timer is canceled if it had been started.

Referring now to the right branch of the flow diagram, at step 1016 the vLP monitors for an atrial event message from the aLP, as was noted above. At step 1018 there is a determination of whether or not the vLP has received an atrial event message from the aLP. If the answer to the determination at step 1018 is No, then flow returns to step 1016. If the answer to the determination at step 1018 is Yes, the flow goes to step 1020. At step 1020 the AV interval timer of the vLP is started, the VV interval timer is canceled, and flow goes to step 1022. An AV interval value used by the AV interval timer can be fixed, or alternatively can be rate dependent. For example, when the AV interval value is rate dependent, an equation or lookup table can be used to determine the AV interval value based on a sensed intrinsic heart rate. The AV interval value used by the AV interval timer can be the same regardless of whether the atrial event message received by the vLP from the aLP at step 1018 indicates that the atrial event was a sensed atrial event or a paced atrial event. Alternatively, AV interval value used by the AV interval timer can be different when the atrial event message received by the vLP from the aLP at step 1018 indicated that the atrial event was a sensed atrial event, compared to when the atrial event message received by the vLP from the aLP at step 1018 indicated that the atrial event was a paced atrial event. An example AV interval value that the AV interval timer may be set to is 200 msec, but is not limited thereto. Still referring to FIG. 10, at step 1022 there is a determination of whether or not the AV interval timer has expired. If the answer to the determination at step 1022 is No, then flow returns to step 1006. If the answer to the determination at step 1022 is Yes, then flow goes to step 1024. At step 1024 the vLP paces the ventricular chamber by delivering one or more pacing stimulation pulses to the ventricular chamber (in or on which the vLP is implanted). Following the vLP pacing the ventricular chamber at step 1024, flow goes to step 1026.

At step 1026 the vLP determines whether or not it has received atrial event messages from the aLP during the two most recent consecutive cardiac cycles. If the answer to the determination at step 1026 is No, then flow returns to step 1004, which was already described above. If the answer to the determination at step 1026 is Yes, then flow goes to step 1028. At step 1028 the vLP determines an interval between instances of the atrial event messages received from the aLP during the two most recent consecutive cardiac cycles. In other words, at step 1028 the vLP determines the duration between when the two most recent atrial event messages that were received from the aLP during the two most recent consecutive cardiac cycles. At step 1030 the vLP redefines its ventricular target interval as being equal to the interval determined at step 1028, i.e., as being equal to the interval between instances of the atrial event messages received from the aLP during the two most recent consecutive cardiac cycles. Flow then returns to step 1004 and the VV interval timer is restarted using the ventricular target interval redefined at step 1030.

In certain embodiments described above, e.g., with reference to FIG. 10, the ventricular target interval is redefined (aka updated) by the vLP itself at instances of step 1030 (in FIG. 10) when an atrial event message has been received by the vLP during the two most recent cardiac cycles. Accordingly, in certain embodiments described above it is the responsibility of the vLP to determine when it is appropriate to redefine (aka update) the ventricular target interval, and it is the responsibility of the vLP to determine the value of the redefined (aka updated) ventricular target interval. In alternative embodiments, it is the responsibility of the aLP to determine the value of the ventricular target interval, and the aLP sends the ventricular target interval to the vLP at least some of the times when the aLP sends atrial event messages to the vLP. For example, referring briefly back to FIGS. 7A and 7C, in accordance with certain embodiments, whenever the aLP transmits an atrial event message to the vLP at an instance of step 718, the aLP transmits a ventricular target interval (or an equivalent) to the vLP within a portion of the atrial event message (e.g., in a payload, header, or footer of the atrial event message). In certain such embodiments, the ventricular target interval included in a portion of the atrial event message (sent from the aLP to the vLP) at an instance of step 718 is equal to the AA interval determined at the most recent instance of step 712.

In the embodiments described above with reference to FIGS. 7A through 7D, the aLP determines at instances of step 716 whether a variation associated with a just determined AA interval is within a specified tolerance. In the specific embodiments described above with reference to FIGS. 7B and 7D, when the variation associated with just determined AA interval is not within a specified tolerance, the aLP sends an i2i message from aLP to vLP for at least two consecutive cardiac cycles, to thereby enable vLP to determine a redefined (aka updated) ventricular target interval at instances of step 1030 in FIG. 10. However, where it is the aLP that determines the ventricular target interval and sends it to the vLP, there is no need for the aLP to send an i2i message from aLP to vLP for at least two consecutive cardiac cycles, which further reduces the aLP's communication burden. Explained another way, the embodiments of FIGS. 7B and 7D are not relevant to the alternative embodiments where it is the aLP's responsibility (rather than the vLP's responsibility) to determine the value of the redefined (aka updated) ventricular target interval.

In the alternative embodiments introduced above where it is the aLP's responsibility (rather than the vLP's responsibility) to determine the value of the redefined (aka updated) ventricular target interval, the steps performed by the vLP (described with reference to FIG. 10) can be simplified. More specifically, steps 1026, 1028 and 1030 in FIG. 10 can be eliminated, and flow can go directly from step 1024 to step 1004 whenever the ventricular chamber is paced at an instance of step 1024 in FIG. 10. In such embodiments, the vLP can receive an update to the ventricular target interval within an atrial event message received by the vLP from the aLP, as part of the vLP monitoring for an atrial event message from the aLP at step 1016 in FIG. 10. Then, as part of step 1020 (or part of another step, or a new step, that occurs after the answer to the determination at step 1018 is Yes), the vLP can store and thereafter use the update to the ventricular target interval that the vLP received from the aLP.

When atrial event messages are sent from the aLP to the vLP, such atrial event messages may or may not include a payload, e.g., depending on whether there is additional information that the aLP wants to send to the vLP (in addition to an indication that an intrinsic atrial event was sensed by the aLP or that a paced atrial event was (or is about to be) caused by the aLP). For a specific example, whenever the aLP wants to inform the vLP of a pacing mode switch, the aLP may include an automatic mode switch (AMS) message in the payload of the atrial event message. In certain embodiments of the present technology, the aLP can send an update to the ventricular target interval to the vLP within a payload of an atrial event message. It may also be possible for an update to the ventricular target interval to instead be sent from the aLP to the vLP within a header or a footer of an atrial event message. Other variations are also possible and within the scope of the embodiments described herein.

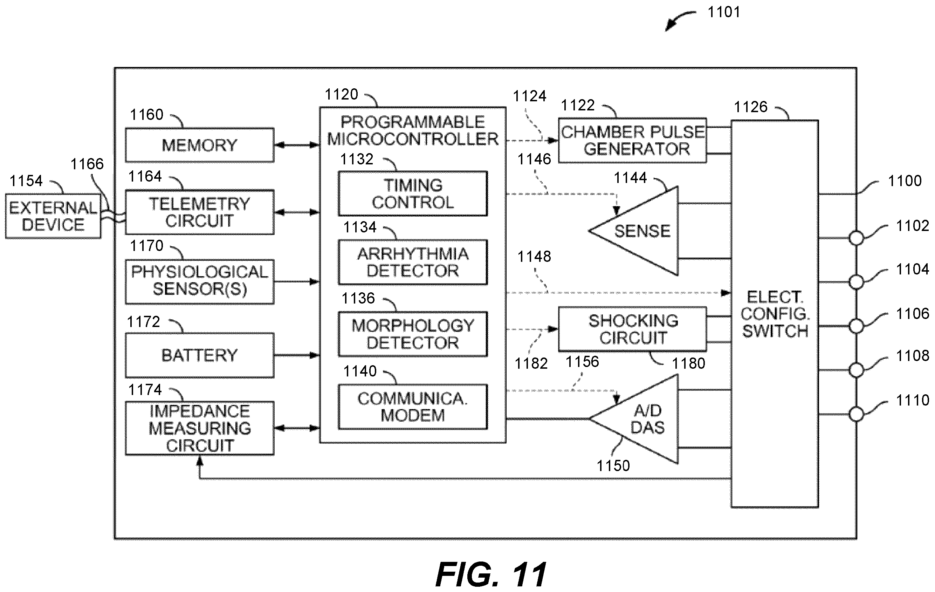

FIG. 11 shows a block diagram of one embodiment of an IMD (e.g., an LP or ICD) 1101 that is implanted into the patient as part of the implantable cardiac system in accordance with certain embodiments herein. Optionally, the IMD 1101 may provide full-function cardiac resynchronization therapy. Alternatively, the IMD 1101 may be implemented with a reduced set of functions and components. For instance, the IMD may be implemented without ventricular sensing and pacing.

The IMD 1101 has a housing 1100 to hold the electronic/computing components. Housing 1100 (which is often referred to as the "can", "case", "encasing", or "case electrode") may be programmably selected to act as the return electrode for certain stimulus modes. Housing 1100 may further include a connector (not shown) with a plurality of terminals 1102, 1104, 1106, 1108, and 1110. The terminals may be connected to electrodes that are located in various locations on housing 1100 or elsewhere within and about the heart. The IMD 1101 includes a programmable microcontroller 1120 that controls various operations of the IMD 1101, including cardiac monitoring and stimulation therapy. Microcontroller 1120 includes a microprocessor (or equivalent control circuitry), RAM and/or ROM memory, logic and timing circuitry, state machine circuitry, and I/O circuitry.

The IMD 1101 further includes a first pulse generator 1122 that generates stimulation pulses for delivery by one or more electrodes coupled thereto. Pulse generator 1122 is controlled by microcontroller 1120 via control signal 1124. Pulse generator 1122 may be coupled to the select electrode(s) via an electrode configuration switch 1126, which includes multiple switches for connecting the desired electrodes to the appropriate I/O circuits, thereby facilitating electrode programmability. Switch 1126 is controlled by a control signal 1128 from microcontroller 1120.

In the embodiment of FIG. 11, a single pulse generator 1122 is illustrated. Optionally, the IMD may include multiple pulse generators, similar to pulse generator 1122, where each pulse generator is coupled to one or more electrodes and controlled by microcontroller 1120 to deliver select stimulus pulse(s) to the corresponding one or more electrodes.

Microcontroller 1120 is illustrated as including timing control circuitry 1132 to control the timing of the stimulation pulses (e.g., pacing rate, atrio-ventricular (AV) delay, atrial interconduction (A-A) delay, or ventricular interconduction (V-V) delay, etc.). Timing control circuitry 1132 may also be used for the timing of refractory periods, blanking intervals, noise detection windows, evoked response windows, alert intervals, marker channel timing, and so on. Microcontroller 1120 also has an arrhythmia detector 1134 for detecting arrhythmia conditions and a morphology detector 1136. Although not shown, the microcontroller 1120 may further include other dedicated circuitry and/or firmware/software components that assist in monitoring various conditions of the patient's heart and managing pacing therapies.

The IMD 1101 is further equipped with a communication modem (modulator/demodulator) 1140 to enable wireless communication with the remote slave pacing unit. Modem 1140 may include one or more transmitters and two or more receivers as discussed herein in connection with FIG. 2. In one implementation, modem 1140 may use low or high frequency modulation. As one example, modem 1140 may transmit i2i messages and other signals through conductive communication between a pair of electrodes. Modem 1140 may be implemented in hardware as part of microcontroller 1120, or as software/firmware instructions programmed into and executed by microcontroller 1120. Alternatively, modem 1140 may reside separately from the microcontroller as a standalone component.

The IMD 1101 includes a sensing circuit 1144 selectively coupled to one or more electrodes, that perform sensing operations, through switch 1126 to detect the presence of cardiac activity in the right chambers of the heart. Sensing circuit 1144 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The automatic gain control enables the unit to sense low amplitude signal characteristics of atrial fibrillation. Switch 1126 determines the sensing polarity of the cardiac signal by selectively closing the appropriate switches. In this way, the clinician may program the sensing polarity independent of the stimulation polarity.

The output of sensing circuit 1144 is connected to microcontroller 1120 which, in turn, triggers or inhibits the pulse generator 1122 in response to the presence or absence of cardiac activity. Sensing circuit 1144 receives a control signal 1146 from microcontroller 1120 for purposes of controlling the gain, threshold, polarization charge removal circuitry (not shown), and the timing of any blocking circuitry (not shown) coupled to the inputs of the sensing circuitry.

In the embodiment of FIG. 11, a single sensing circuit 1144 is illustrated. Optionally, the IMD may include multiple sensing circuits, similar to sensing circuit 1144, where each sensing circuit is coupled to one or more electrodes and controlled by microcontroller 1120 to sense electrical activity detected at the corresponding one or more electrodes. Sensing circuit 1144 may operate in a unipolar sensing configuration or in a bipolar sensing configuration.

IMD 1101 further includes an analog-to-digital (A/D) data acquisition system (DAS) 1150 coupled to one or more electrodes via switch 1126 to sample cardiac signals across any pair of desired electrodes. Data acquisition system 1150 is configured to acquire intracardiac electrogram signals, convert the raw analog data into digital data, and store the digital data for later processing and/or telemetric transmission to an external device 1154 (e.g., a programmer, local transceiver, or a diagnostic system analyzer). Data acquisition system 1150 is controlled by a control signal 1156 from the microcontroller 1120.

Microcontroller 1120 is coupled to a memory 1160 by a suitable data/address bus. The programmable operating parameters used by microcontroller 1120 are stored in memory 1160 and used to customize the operation of IMD 1101 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart within each respective tier of therapy.

The operating parameters of IMD 1101 may be non-invasively programmed into memory 1160 through a telemetry circuit 1164 in telemetric communication via communication link 1166 with external device 1154. Telemetry circuit 1164 allows intracardiac electrograms and status information relating to the operation of IMD 1101 (as contained in microcontroller 1120 or memory 1160) to be sent to external device 1154 through communication link 1166.

The IMD 1101 can further include magnet detection circuitry (not shown), coupled to microcontroller 1120, to detect when a magnet is placed over the unit. A magnet may be used by a clinician to perform various test functions of IMD 1101 and/or to signal microcontroller 1120 that external device 1154 is in place to receive or transmit data to microcontroller 1120 through telemetry circuits 1164.

The IMD 1101 can further include one or more physiological sensors 1170. Such sensors are commonly referred to as "rate-responsive" sensors because they are typically used to adjust pacing stimulation rates according to the exercise state of the patient. However, physiological sensor 1170 may further be used to detect changes in cardiac output, changes in the physiological condition of the heart, or diurnal changes in activity (e.g., detecting sleep and wake states). Signals generated by physiological sensors 1170 are passed to microcontroller 1120 for analysis. Microcontroller 1120 responds by adjusting the various pacing parameters (such as rate, AV Delay, V-V Delay, etc.) at which the atrial and ventricular pacing pulses are administered. While shown as being included within IMD 1101, physiological sensor(s) 1170 may be external to IMD 1101, yet still be implanted within or carried by the patient. Examples of physiologic sensors might include sensors that, for example, sense respiration rate, pH of blood, ventricular gradient, activity, position/posture, minute ventilation (MV), and so forth.

A battery 1172 provides operating power to all of the components in IMD 1101. Battery 1172 is capable of operating at low current drains for long periods of time, and is capable of providing high-current pulses (for capacitor charging) when the patient requires a shock pulse (e.g., in excess of 2 A, at voltages above 2 V, for periods of 10 seconds or more). Battery 1172 also desirably has a predictable discharge characteristic so that elective replacement time can be detected. As one example, IMD 1101 employs lithium/silver vanadium oxide batteries.

IMD 1101 further includes an impedance measuring circuit 1174, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. Impedance measuring circuit 1174 is coupled to switch 1126 so that any desired electrode may be used. In this embodiment IMD 1101 further includes a shocking circuit 1180 coupled to microcontroller 1120 by a data/address bus 1182.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in which the pacemaker is implanted (and thus with which the LP is interacting; e.g., pacing and/or sensing). Some non-limiting examples include sensing sensitivity, an evoked response algorithm, use of AF suppression in a local chamber, blanking & refractory periods, etc. Accordingly, each LP needs to know an identity of the chamber in which the LP is implanted, and processes may be implemented to automatically identify a local chamber associated with each LP.

An aspect of the embodiments described herein relates to an atrial leadless pacemaker (aLP) configured to be implanted in or on an atrial chamber, and a ventricular leadless pacemaker (vLP) configured to be implanted in or on a ventricular chamber. The aLP is configured to determine an atrial-to-atrial interval (AA interval) for an atrial event, wherein the AA interval comprises a duration between the atrial event and an immediately preceding atrial event. Additionally, the aLP is configured to determine whether or not a variation associated with the AA interval is within a specified tolerance, and based thereon, determine whether or not an atrial event message should be transmitted to the vLP to inform the vLP of the atrial event. The aLP is also configured to abstain from transmitting the atrial event message to the vLP, when there is a determination that the atrial event message should not be transmitted to the vLP, to thereby abstain from informing the vLP of the atrial event. Further, the aLP is configured to transmit the atrial event message to the vLP, when there is a determination that the atrial event message should be transmitted to the vLP, to thereby inform the vLP of the atrial event.

In an embodiment, the aLP is configured to: determine that the atrial event message should not be transmitted to the vLP when the AA interval is determined to be within specified tolerance; and determine that the atrial event message should be transmitted to the vLP when the AA interval is not determined to be within specified tolerance.

In an embodiment, the aLP is configured to: determine that the atrial event message should not be transmitted to the vLP when the AA interval and an immediately preceding AA interval are each within specified tolerance; and determine that the atrial event message should be transmitted to the vLP when at least one of the AA interval or the immediately preceding AA interval is not within specified tolerance.

In an embodiment, the aLP is configured to: determine whether or not a respective atrial event message should be transmitted to the vLP for intrinsic atrial events sensed by the aLP; and determine that a respective atrial event message should be transmitted to the vLP for paced atrial events caused by the aLP.

In an embodiment, the aLP is configured to determine whether or not a respective atrial event message should be transmitted to the vLP for intrinsic atrial events sensed by the aLP and for paced atrial events caused by the aLP.

In an embodiment, the vLP is configured to: use a ventricular-to-ventricular interval (VV interval) timer to determine whether or not a target ventricular interval has elapsed since a most recent ventricular event has occurred; and deliver pacing stimulation to the ventricular chamber, in response to the VV interval timer expiring without the vLP receiving an atrial event message from the aLP during the target ventricular interval.

In an embodiment, the vLP is configured to set the VV interval timer to a ventricular target interval when the VV interval timer is reset. In certain such embodiments, the vLP is configured to determine whether or not instances of the atrial event message were received from the aLP during two consecutive most recent cardiac cycles; and when instances of the atrial event message were received from the aLP during the two consecutive most recent cardiac cycles, update the ventricular target interval based on an interval between when the instances of the atrial event message were received from the aLP during the two consecutive most recent cardiac cycles. In other embodiments, an update to the ventricular target interval is determined by the aLP and transmitted to the vLP within an atrial event message, which eliminates the need for the vLP to determine whether or not instances of the atrial event message were received from the aLP during two consecutive most recent cardiac cycles, and also eliminates the need for the vLP to determine an interval between when the instances of the atrial event message were received from the aLP during the two consecutive most recent cardiac cycles.

In an embodiment, the vLP is configured to: use an atrioventricular interval (AV interval) timer to determine when a specified AV interval has elapsed since a most recent atrial event has occurred; start the AV interval timer and cancel the VV interval timer in response to receiving an atrial event message from the aLP; and deliver pacing stimulation to the ventricular chamber in response to the AV interval timer expiring.

In an embodiment, the aLP is configured to determine whether or not the variation associated with an AA interval is within the specified tolerance by: determining a running delta indicative of a cumulative difference between each consecutive pair of AA intervals determined since the aLP most recently transmitted the atrial event message to the vLP; and determining that the variation associated with the AA interval is within the specified tolerance when the running delta is within the specified tolerance.

In an embodiment, the aLP is configured to determine whether or not the variation associated with an AA interval is within the specified tolerance by: determining a cumulative interval between the AA interval and an AA interval corresponding to when the aLP most recently transmitted the atrial event message to the vLP; determining, based on the cumulative interval and the AA interval, a deviation value indicative of how much AA intervals occurring during the cumulative interval deviate from the AA interval corresponding to when the aLP most recently transmitted the atrial event message to the vLP; and determining that the variation associated with the AA interval is within the specified tolerance when the deviation value is within the specified tolerance.

In an embodiment, the aLP is configured to determine whether or not the variation associated with an AA interval is within the specified tolerance by determining a delta between the AA interval and an AA interval corresponding to an immediately preceding atrial event, and determining that the variation associated with the AA interval is within the specified tolerance when the delta is within the specified tolerance.

In an embodiment, the aLP abstaining from transmitting the atrial event message to the vLP reduces an amount of power consumed by the aLP compared to when the aLP transmits the atrial event message to the vLP.

In an embodiment, the aLP is configured to use conductive communication to transmit the atrial event messages, and other types of messages, to the vLP. In other embodiments, the aLP is configured to use radio frequency (RF) communication or inductive communication when to transmit the atrial event messages, and other types of messages, to the vLP.

This aspect of the embodiments also relates to methods for use by a dual chamber leadless pacemaker system including an atrial leadless pacemaker (aLP) implanted in or on an atrial chamber and a ventricular leadless pacemaker (vLP) implanted in or on a ventricular chamber. Such a method can include the aLP determining an atrial-to-atrial interval (AA interval) for an atrial event and determining whether or not a variation associated with the AA interval is within a specified tolerance, wherein the AA interval comprises a duration between the atrial event and an immediately preceding atrial event. The method can also include the aLP determining, based on results of the determining whether or not the variation associated with the AA interval is within the specified tolerance, whether or not an atrial event message should be transmitted to the vLP to inform the vLP of the atrial event. The method can further include the aLP, in response to determining that the atrial event message should not be transmitted to the vLP, abstaining from transmitting the atrial event message to the vLP and thereby abstaining from informing the vLP of the atrial event.

In an embodiment, the aLP determining whether or not the atrial event message should be transmitted to the vLP comprises the aLP determining that the atrial event message should not be transmitted to the vLP when the AA interval is determined to be within specified tolerance.

In an embodiment, the aLP determining whether or not the atrial event message should be transmitted to the vLP comprises: the aLP determining whether or not a variation associated with an immediately preceding AA interval was within the specified tolerance; the aLP determining that the atrial event message should not be transmitted to the vLP when the AA interval and the immediately preceding AA interval are each within specified tolerance; and the aLP determining that the atrial event message should be transmitted to the vLP when at least one of the AA interval or the immediately preceding AA interval is not within specified tolerance.

In an embodiment, the atrial event and the immediately preceding atrial event each comprise an intrinsic atrial event sensed by the aLP. In accordance with other embodiments, the atrial event and the immediately preceding atrial event each comprise either one of an intrinsic atrial event sensed by the aLP or a paced atrial event caused by the aLP.

In an embodiment, the method also includes the aLP determining a further AA interval when a further atrial event occurs and determining whether or not a variation associated with the further AA interval is within the specified tolerance, wherein the further AA interval corresponds to a duration between the further atrial event and an immediately preceding atrial event. The method also includes the aLP, in response to determining that the variation associated with the further AA interval is not within the specified tolerance, transmitting an atrial event message to the vLP and thereby informing the vLP of the further atrial event.

In an embodiment, the vLP uses a ventricular-to-ventricular interval (VV interval) timer to determine whether or not a target ventricular interval has elapsed since a most recent ventricular event has occurred. In certain such embodiments, the method further comprises the vLP, in response to the VV interval timer expiring without the vLP receiving an atrial event message from the aLP during the target ventricular interval, delivering pacing stimulation to the ventricular chamber. In accordance with certain embodiments, the vLP sets the VV interval timer to a ventricular target interval when the VV interval timer is reset.

In an embodiment, where it is the vLP's responsibility to selectively update the target ventricular interval, the vLP, in response to receiving the atrial event message from the aLP, determines whether or not the vLP also received an atrial event message from the aLP during an immediately preceding cardiac cycle and thus received instances of the atrial event message from the aLP during two consecutive most recent cardiac cycles. Such a method can also include the vLP, in response to receiving the instances of the atrial event message from the aLP during the two consecutive most recent cardiac cycles, updating the ventricular target interval based on an interval between when the instances of the atrial event message were received from the aLP during the two consecutive most recent cardiac cycles. In accordance with certain embodiments, the vLP cancels the VV interval timer in response to the vLP receiving the atrial event message from the aLP.

In an embodiment, where it is the aLP's responsibility (instead of the vLP's responsibility) to update the target ventricular interval, the atrial event message transmitted by the aLP to the vLP includes an update to the ventricular target interval determined by the aLP.

In an embodiment, the vLP uses an atrioventricular interval (AV interval) timer to determine when a specified AV interval has elapsed since a most recent atrial event has occurred. In certain such embodiments, the vLP receives the atrial event message from the aLP, and in response thereto, starts the AV interval timer. In accordance with certain embodiments, the vLP delivers pacing stimulation to the ventricular chamber in response to the AV interval timer expiring.

In an embodiment, the aLP determining whether or not the variation associated with the AA interval is within the specified tolerance, comprises the aLP: determining a running delta indicative of a cumulative difference between each consecutive pair of AA intervals determined since the aLP most recently transmitted the atrial event message to the vLP; and determining that the variation associated with the AA interval is within the specified tolerance when the running delta is within the specified tolerance.

In an embodiment, the aLP determining whether or not the variation associated with the AA interval is within the specified tolerance, comprises the aLP: determining a cumulative interval between the AA interval and an AA interval corresponding to when the aLP most recently transmitted the atrial event message to the vLP; determining, based on the cumulative interval and the AA interval, a deviation value indicative of how much AA intervals occurring during the cumulative interval deviate from the AA interval corresponding to when the aLP most recently transmitted the atrial event message to the vLP; and determining that the variation associated with the AA interval is within the specified tolerance when the deviation value is within the specified tolerance.

In an embodiment, the aLP determining whether or not the variation associated with the AA interval is within the specified tolerance, comprises the aLP: determining a delta between the AA interval and an AA interval corresponding to an immediately preceding atrial event; and determining that the variation associated with the AA interval is within the specified tolerance when the delta is within the specified tolerance.

In an embodiment, the aLP abstaining from transmitting the atrial event message to the vLP reduces an amount of power consumed by the aLP compared to when the aLP transmits the atrial event message to the vLP.

In an embodiment, the aLP uses conductive communication when transmitting the atrial event message, and other types of messages, to the vLP. In other embodiments, the aLP uses radio frequency (RF) communication or inductive communication when transmitting the atrial event message, and other types of messages, to the vLP.

In an embodiment, a method includes an aLP sensing intrinsic atrial events and for each intrinsic atrial event that is sensed determining a respective atrial-to-atrial interval (AA interval) that corresponds to a duration between the intrinsic atrial event and an immediately preceding intrinsic atrial event. The method further includes the aLP, based on the determined AA intervals, selectively transmitting and selectively abstaining from transmitting atrial event messages to the vLP. In accordance with certain embodiments, the aLP determines, for each intrinsic atrial event of at least some of the intrinsic atrial events, whether or not a variation associated with the AA interval corresponding to the intrinsic atrial event is within a specified tolerance, and in dependence thereon the aLP determines whether or not the aLP should transmit an atrial event message to the vLP. In certain such embodiments, the aLP abstains from transmitting the atrial event message to the vLP when the AA interval corresponding to an intrinsic atrial event is within the specified tolerance and a further AA interval corresponding to an immediately preceding intrinsic atrial event is also within the specified tolerance.

This aspect of the invention relates to an atrial leadless pacemaker (aLP) configured to be implanted in or on an atrial chamber, wherein the aLP comprises a sense circuit, a transmitter, a pair of electrodes, and a controller. The sense circuit is configured to sense a signal indicative of cardiac activity from which atrial events can be detected. The pair of electrodes is configured to deliver stimulation pulses to the atrial chamber. The controller, which is communicatively coupled to the sense circuit and the transmitter, is configured to determine an atrial-to-atrial interval (AA interval) for an atrial event, wherein the AA interval comprises a duration between the atrial event and an immediately preceding atrial event. The controller is also configured to determine whether or not a variation associated with the AA interval is within a specified tolerance, and based thereon, determine whether or not an atrial event message should be transmitted to the vLP to inform the vLP of the atrial event. Additionally, the controller is configured to cause the transmitter to transmit the atrial event message to the vLP, when there is a determination that the atrial event message should be transmitted to the vLP, to thereby inform the vLP of the atrial event. The controller is further configured to abstain from causing the transmitter to transmit the atrial event message to the vLP, when there is a determination that the atrial event message should not be transmitted to the vLP, to thereby abstain from informing the vLP of the atrial event.

In an embodiment, the aLP includes one or more pulse generators, at least one of which is used to provide pulses to the pair of electrodes to thereby cause the pair of electrodes to deliver stimulation pulses to the atrial chamber under control of the controller.

In an embodiment, the transmitter comprises a conductive communication transmitter including at least one of the one or more pulse generators that provides pulses to the pair of electrodes, under control of the controller, to thereby cause the pair of electrodes to output conductive communication pulses encoded to include the atrial event message. Alternatively, the transmitter can comprise one of a radio frequency (RF) communication transmitter or an inductive communication transmitter, and the atrial event message can be transmitted as an RF signal or an inductive signal.

In an embodiment, the sense circuit comprises a sense amplifier coupled to the pair of electrodes, and the signal indicative of cardiac activity comprises an electrogram (EGM) indicative of cardiac electrical activity. In accordance with other embodiments, the sense circuit comprises an accelerometer or a microphone, and the signal indicative of cardiac activity comprises a heart sounds signal.

In an embodiment, the aLP comprises a battery that powers the aLP, and power consumption is reduced when the controller abstains from causing the transmitter to transmit the atrial event message to the vLP, compared to when the controller causes the transmitter to transmit the atrial event message to the vLP.

This aspect of the invention relates to a ventricular leadless pacemaker (vLP) configured to be implanted in or on a ventricular chamber, wherein the vLP comprises a sense circuit, a receiver, a pulse generator, a pair of electrodes, and a controller. The sense circuit is configured to sense a signal indicative of cardiac activity from which ventricular events can be detected. The pair of electrodes is configured to deliver stimulation pulses output by the pulse generator to the ventricular chamber. The controller is communicatively coupled to the sense circuit, the pulse generator, and the receiver. An atrioventricular interval (AV interval) timer is used to determine whether a specified AV interval has elapsed since a most recent atrial event, and a ventricular-to-ventricular interval (VV interval) timer is used to determine whether a target ventricular interval has elapsed since a most recent ventricular event. At least one of the AV interval timer and the VV interval timer can be implemented by the controller, but that need not be the case. The controller of the vLP is configured to start the AV interval timer when an atrial event message is received by the vLP from an atrial leadless pacemaker (aLP) configured to be implanted in or on an atrial chamber. The controller of the vLP is also configured to cause to the pulse generator to provide a stimulation pulse to the pair of electrodes, when the AV interval timer expires, to thereby cause the ventricular chamber to be paced by the vLP at approximately the AV interval following an immediately preceding atrial event. Additionally, the controller of the vLP is configured to set the VV interval timer to a ventricular target interval when the VV interval timer is reset, start the VV interval timer in response to a ventricular event, and cause to the pulse generator to provide a stimulation pulse to the pair of electrodes and cause the VV interval timer to be reset, when the VV interval timer expires without the receiver receiving the atrial event message from the aLP, to thereby cause the ventricular chamber to be paced at approximately the ventricular target interval following an immediately preceding ventricular event. Additionally, the controller of the vLP is configured to cancel the VV interval timer, when the vLP receives the atrial event message from the aLP.

In an embodiment, where it is the vLP's responsibility to selectively update the ventricular target interval, the controller of the vLP is also configured to determine whether the atrial event message was also received from the aLP during an immediately preceding cardiac cycle and thus instances of the atrial event message were received from the aLP during two consecutive most recent cardiac cycles. Further, when the instances of the atrial event message were received from the aLP during two consecutive most recent cardiac cycles, the controller of the vLP is configured to update the ventricular target interval based on an interval between when the instances of the atrial event message were received from the aLP during the two consecutive most recent cardiac cycles.

In an embodiment, where it is the aLP's responsibility (instead of the vLP's responsibility) to update the target ventricular interval, the atrial event message transmitted by the aLP to the vLP, and received by the vLP, includes an update to the ventricular target interval that was determined by the aLP. In such alternative embodiments, the vLP does not need to determine updates to the ventricular target interval from two consecutive atrial event messages received from aLP. Instead the aLP can explicitly inform the vLP of a new value for (i.e., an update to) the ventricular target interval via a single atrial event message. In certain such embodiments, the new value for (i.e., an update to) the ventricular target interval, which is determined by the aLP, is equal to the AA interval most recently determined by the aLP.

Embodiments of the present technology have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope and spirit of the claimed invention. For example, it would be possible to combine or separate some of the steps shown in FIGS. 7A-7D and in FIGS. 8 through 10. It would also be possible to reorder some of the steps shown in FIGS. 7A-7D and in FIGS. 8 through 10. For another example, it is possible to change the boundaries of some of the blocks shown in FIGS. 2 and 11.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the invention is defined by the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

**Claims**

1.  An implantable system (100), comprising:

    an atrial leadless pacemaker (aLP) (102a) configured to be implanted in or on an atrial chamber; and
    a ventricular leadless pacemaker (vLP) (102b) configured to be implanted in or on a ventricular chamber;
    the aLP (102a) configured to:
    determine an atrial-to-atrial interval (AA interval) for an atrial event, wherein the AA interval comprises a duration between the atrial event and an immediately preceding atrial event;

    **characterized in that** the aLP (102a) is further configured to:
    determine whether or not a variation associated with the AA interval is within a specified tolerance, and based thereon, determine whether or not an atrial event message should be transmitted to the vLP to inform the vLP of the atrial event;

abstain from transmitting the atrial event message to the vLP, when there is a determination that the atrial event message should not be transmitted to the vLP, to thereby abstain from informing the vLP of the atrial event; and transmit the atrial event message to the vLP, when there is a determination that the atrial event message should be transmitted to the vLP, to thereby inform the vLP of the atrial event.

2. The implantable system (100) of claim 1, wherein the aLP (102a) is configured to:

   determine that the atrial event message should not be transmitted to the vLP (102b) when the AA interval is determined to be within the specified tolerance; and
   determine that the atrial event message should be transmitted to the vLP (102b) when the AA interval is not determined to be within the specified tolerance.

3. The implantable system (100) of claim 1, wherein the aLP (102a) is configured to:

   determine that the atrial event message should not be transmitted to the vLP (102b) when the AA interval and an immediately preceding AA interval are each within the specified tolerance; and
   determine that the atrial event message should be transmitted to the vLP (102b) when at least one of the AA interval or the immediately preceding AA interval is not within the specified tolerance.

4. The implantable system (100) of any one of claims 1 through 3, wherein the aLP (102a) is configured to:

   determine whether or not a respective said atrial event message should be transmitted to the vLP (102b) for intrinsic atrial events sensed by the aLP (102a); and
   determine that a respective said atrial event message should be transmitted to the vLP (102b) for paced atrial events caused by the aLP (102a).

5. The implantable system (100) of any one of claims 1 through 3, wherein the aLP (102a) is configured to determine whether or not a respective said atrial event message should be transmitted to the vLP (102b) for intrinsic atrial events sensed by the aLP (102a) and for paced atrial events caused by the aLP (102a).

6. The implantable system (100) of any one of claims 1 through 5, wherein the vLP (102b) is configured to:

   use a ventricular-to-ventricular interval (VV interval) timer (115) to determine whether or not a ventricular target interval has elapsed since a most recent ventricular event has occurred; and
   set the VV interval timer (115) to the ventricular target interval when the VV interval timer (115) is reset.

7. The implantable system (100) of claim 6, wherein the aLP (102a) is configured to include an update to the ventricular target interval within the atrial event message transmitted by the aLP (102a) to the vLP (102b).

8. The implantable system (100) of any one of claims 6 or 7, wherein the vLP (102b) is configured to:
   deliver pacing stimulation to the ventricular chamber, in response to the VV interval timer (115) expiring without the vLP (102b) receiving an atrial event message from the aLP (102a) during the target ventricular interval.

9. The implantable system (100) of any one of claims 6 through 8, wherein the vLP (102b) is configured to:

   determine whether or not instances of the atrial event message were received from the aLP (102a) during two consecutive most recent cardiac cycles; and
   when instances of the atrial event message were received from the aLP (102a) during the two consecutive most recent cardiac cycles, update the ventricular target interval based on an interval between when the instances of the atrial event message were received from the aLP (102a) during the two consecutive most recent cardiac cycles.

10. The implantable system (100) of any one of claims 6 through 9, wherein the vLP (102b) is configured to:

   use an atrioventricular interval (AV interval) timer (115) to determine when a specified AV interval has elapsed since a most recent atrial event has occurred;
   start the AV interval timer and cancel the VV interval timer (115) in response to receiving a said atrial event message from the aLP (102a); and

deliver pacing stimulation to the ventricular chamber in response to the AV interval timer (115) expiring.

11. The implantable system (100) of any one of claims 1 through 10, wherein the aLP (102a) is configured to determine whether or not the variation associated with a said AA interval is within the specified tolerance by:

   determining a running delta indicative of a cumulative difference between each consecutive pair of AA intervals determined since the aLP (102a) most recently transmitted the atrial event message to the vLP (102b); and determining that the variation associated with the AA interval is within the specified tolerance when the running delta is within the specified tolerance.

12. The implantable system (100) of any one of claims 1 through 10, wherein the aLP (102a) is configured to determine whether or not the variation associated with a said AA interval is within the specified tolerance by:

   determining a cumulative interval between the AA interval and an AA interval corresponding to when the aLP (102a) most recently transmitted the atrial event message to the vLP (102b);
   determining, based on the cumulative interval and the AA interval, a deviation value indicative of how much AA intervals occurring during the cumulative interval deviate from the AA interval corresponding to when the aLP (102a) most recently transmitted the atrial event message to the vLP (102b); and
   determining that the variation associated with the AA interval is within the specified tolerance when the deviation value is within the specified tolerance.

13. The implantable system (100) of any one of claims 1 through 10, wherein the aLP (102a) is configured to determine whether or not the variation associated with a said AA interval is within the specified tolerance by:

   determining a delta between the AA interval and an AA interval corresponding to an immediately preceding atrial event; and
   determining that the variation associated with the AA interval is within the specified tolerance when the delta is within the specified tolerance.

14. The implantable system (100) of any one of claims 1 through 13, wherein the aLP (102a) abstaining from transmitting the atrial event message to the vLP (102b) reduces an amount of power consumed by the aLP (102a) compared to when the aLP (102a) transmits the atrial event message to the vLP (102b).

15. The implantable system (100) of any one of claims 1 through 14, wherein the aLP (102a) is configured to use conductive communication, radio frequency (RF) communication, or inductive communication to transmit the atrial event messages, and other types of messages, to the vLP.

**Patentansprüche**

1. Implantierbares System (100), umfassend:

   einen atrialen kabellosen Herzschrittmacher (aLP) (102a), der dazu konfiguriert ist, in oder an einer Herzvorkammer implantiert zu werden; und
   einen ventrikulären kabellosen Herzschrittmacher (vLP) (102), der dazu konfiguriert ist, in oder an eine Herzkammer implantiert zu werden;
   wobei der aLP (102a) zu Folgendem konfiguriert ist:

   Bestimmen eines Vorkammer-zu-Vorkammer-Intervalls (AA-Intervalls) für ein Vorkammerereignis, wobei das AA-Intervall eine Dauer zwischen dem Vorkammerereignis und einem unmittelbar folgenden Vorkammerereignis umfasst;
   **dadurch gekennzeichnet, dass** der aLP (102a) ferner zu Folgendem konfiguriert ist:

   Bestimmen, ob eine dem AA-Intervall zugeordnete Variation innerhalb einer spezifizierten Toleranz liegt oder nicht, und basierend darauf Bestimmen, ob eine Vorkammerereignisnachricht an den vLP zum Informieren des vLP über das Vorkammerereignis übertragen werden sollte;
   Unterlassen des Übertragens der Vorkammerereignisnachricht an den vLP, wenn eine Bestimmung vorliegt, dass die Vorkammerereignisnachricht nicht an den vLP übertragen werden sollte, um somit das Informieren des vLP über das Vorkammerereignis zu unterlassen; und
Übertragen der Vorkammerereignisnachricht an den vLP, wenn eine Bestimmung vorliegt, dass die Vorkammerereignisnachricht an den vLP übertragen werden sollte, um somit den vLP über das Vorkammerereignis zu informieren.

2. Implantierbares System (100) nach Anspruch 1, wobei der aLP (102a) zu Folgendem konfiguriert ist:

Bestimmen, dass die Vorkammerereignisnachricht nicht an den vLP (102b) übertragen werden sollte, wenn bestimmt wird, dass das AA-Intervall innerhalb der spezifizierten Toleranz liegt; und
Bestimmen, dass die Vorkammerereignisnachricht an den vLP (102b) übertragen werden sollte, wenn bestimmt wird, dass das AA-Intervall nicht innerhalb der spezifizierten Toleranz liegt.

3. Implantierbares System (100) nach Anspruch 1, wobei der aLP (102a) zu Folgendem konfiguriert ist:

Bestimmen, dass die Vorkammerereignisnachricht nicht an den vLP (102b) übertragen werden sollte, wenn das AA-Intervall und ein unmittelbar folgendes AA-Intervall jeweils innerhalb der spezifizierten Toleranz liegen; und
Bestimmen, dass die Vorkammerereignisnachricht an den vLP (102b) übertragen werden sollte, wenn mindestens eines des AA-Intervalls oder des unmittelbar folgenden AA-Intervalls nicht innerhalb der spezifizierten Toleranz liegt.

4. Implantierbares System (100) nach einem der Ansprüche 1 bis 3, wobei der aLP (102a) zu Folgendem konfiguriert ist:

Bestimmen, ob für intrinsische Vorkammerereignisse, die durch den aLP (102a) erfasst werden, die jeweilige Vorkammerereignisnachricht an den vLP (102b) übertragen werden sollte oder nicht; und
Bestimmen, dass für Vorkammerschrittmacherereignisse, die durch den aLP (102a) verursacht werden, die jeweilige Vorkammerereignisnachricht an den vLP (102b) übertragen werden sollte.

5. Implantierbares System (100) nach einem der Ansprüche 1 bis 3, wobei der aLP (102a) dazu konfiguriert ist, zu bestimmen, ob für intrinsische Vorkammerereignisse, die durch den aLP (102a) erfasst werden, und für Vorkammerschrittmacherereignisse, die durch den aLP (102a) verursacht werden, die jeweilige Vorkammerereignisnachricht an den vLP (102b) übertragen werden sollte oder nicht.

6. Implantierbares System (100) nach einem der Ansprüche 1 bis 5, wobei der vLP (102b) zu Folgendem konfiguriert ist:

Verwenden eines Zeitgebers (115) für Kammer-zu-Kammer-Intervalle (VV-Intervalle), um zu bestimmen, ob seit einem zuletzt aufgetretenen Kammerereignis ein Kammersollintervall verstrichen ist oder nicht; und
Einstellen des VV-Intervallzeitgebers (115) auf das Kammersollintervall, wenn der VV-Intervallzeitgeber (115) zurückgesetzt wird.

7. Implantierbares System (100) nach Anspruch 6, wobei der aLP (102a) dazu konfiguriert ist, in die Vorkammerereignisnachricht, die durch den aLP (102a) an den vLP (102b) übertragen wird, eine Aktualisierung des Kammersollintervalls einzubeziehen.

8. Implantierbares System (100) nach Anspruch 6 oder 7, wobei der vLP (102b) zu Folgendem konfiguriert ist:

Abgeben von Schrittmacherstimulation an die Herzkammer als Reaktion darauf, dass der VV-Intervallzeitgeber (115) abläuft,
ohne dass der vLP (102b) während des Kammersollintervalls eine Vorkammerereignisnachricht von dem aLP (102a) empfängt.

9. Implantierbares System (100) nach einem der Ansprüche 6 bis 8, wobei der vLP (102b) zu Folgendem konfiguriert ist:

Bestimmen, ob während zwei aufeinanderfolgenden zuletzt aufgetretenen Herzzyklen Fälle der Vorkammerereignisnachricht von dem aLP (102a) empfangen wurden; und
wenn während zwei aufeinanderfolgenden zuletzt aufgetretenen Herzzyklen Fälle der Vorkammerereignisnachricht von dem aLP (102a) empfangen wurden, Aktualisieren des Kammersollintervalls basierend auf einem Intervall zwischen den Zeitpunkten, zu denen die Fälle der Vorkammerereignisnachricht von dem aLP (102a) während den zwei aufeinanderfolgenden zuletzt aufgetretenen Herzzyklen empfangen wurden.

**10.** Implantierbares System (100) nach einem der Ansprüche 6 bis 9, wobei der vLP (102b) zu Folgendem konfiguriert ist:

Verwenden eines Zeitgebers (115) für atrioventrikuläre Intervalle (AV-Intervalle), um zu bestimmen, wann seit einem zuletzt aufgetretenen Vorkammerereignis ein spezifiziertes AV-Intervall verstrichen ist;
Starten des AV-Intervallzeitgebers und Abbrechen des VV-Intervallzeitgebers (115) als Reaktion auf Empfangen der Vorkammerereignisnachricht von dem aLP (102a); und
Abgeben von Schrittmacherstimulation an die Herzkammer als Reaktion darauf, dass der AV-Intervallzeitgeber (115) abläuft.

**11.** Implantierbares System (100) nach einem der Ansprüche 1 bis 10, wobei der aLP (102a) dazu konfiguriert ist, durch Folgendes zu bestimmen, ob die dem AA-Intervall zugeordnete Variation innerhalb der spezifizierten Toleranz liegt oder nicht:

Bestimmen eines laufenden Deltas, das eine kumulative Differenz zwischen jedem aufeinanderfolgenden Paar von AA-Intervallen angibt, die bestimmt wurden, seit die aLP (102a) zuletzt die Vorkammerereignisnachricht an den vLP (102b) übertragen hat; und
Bestimmen, dass die dem AA-Intervall zugeordnete Variation innerhalb der spezifizierten Toleranz liegt, wenn das laufende Delta innerhalb der spezifizierten Toleranz liegt.

**12.** Implantierbares System (100) nach einem der Ansprüche 1 bis 10, wobei der aLP (102a) dazu konfiguriert ist, durch Folgendes zu bestimmen, ob die dem AA-Intervall zugeordnete Variation innerhalb der spezifizierten Toleranz liegt oder nicht:

Bestimmen eines kumulativen Intervalls zwischen dem AA-Intervall und einem AA-Intervall, das dem Zeitpunkt entspricht, zu dem der aLP (102a) zuletzt die Vorkammerereignisnachricht an den vLP (102b) übertragen hat;
Bestimmen, basierend auf dem kumulativen Intervall und dem AA-Intervall, eines Abweichungswerts, der angibt, wieviel AA-Intervalle, die während des kumulativen Intervalls auftreten, von dem AA-Intervall, das dem Zeitpunkt entspricht, zu dem der aLP (102a) zuletzt die Vorkammerereignisnachricht an den vLP (102b) übertragen hat, abweichen; und
Bestimmen, dass die dem AA-Intervall zugeordnete Variation innerhalb der spezifizierten Toleranz liegt, wenn der Abweichungswert innerhalb der spezifizierten Toleranz liegt.

**13.** Implantierbares System (100) nach einem der Ansprüche 1 bis 10, wobei der aLP (102a) dazu konfiguriert ist, durch Folgendes zu bestimmen, ob das AA-Intervall innerhalb der spezifizierten Toleranz liegt oder nicht:

Bestimmen eines Deltas zwischen dem AA-Intervall und einem AA-Intervall, das einem unmittelbar folgenden Vorkammerereignis entspricht; und
Bestimmen, dass die dem AA-Intervall zugeordnete Variation innerhalb der spezifizierten Toleranz liegt, wenn das Delta innerhalb der spezifizierten Toleranz liegt.

**14.** Implantierbares System (100) nach einem der Ansprüche 1 bis 13, wobei der aLP (102a), der das Übertragen der Vorkammerereignisnachricht an den vLP (102b) unterlässt, die Menge an verbrauchter Energie durch den aLP (102a) im Vergleich zu dem aLP (102a), der die Vorkammerereignisnachricht an den vLP (102b) überträgt, reduziert.

**15.** Implantierbares System (100) nach einem der Ansprüche 1 bis 14, wobei der aLP (102a) dazu konfiguriert ist, leitende Kommunikation, Hochfrequenzkommunikation (RF-Kommunikation) oder induktive Kommunikation zu verwenden, um die Vorkammerereignisnachrichten und andere Nachrichtentypen an den vLP zu übertragen.

**Revendications**

**1.** Système implantable (100), comprenant :

un stimulateur cardiaque auriculaire sans fil (aLP) (102a) configuré pour être implanté dans ou sur une cavité auriculaire ; et
un stimulateur cardiaque ventriculaire sans sonde (vLP) (102b) configuré pour être implanté dans ou sur une cavité ventriculaire ;
l'aLP (102a) configuré pour :

déterminer un intervalle auriculaire à auriculaire (intervalle AA) pour un événement auriculaire, dans lequel l'intervalle AA comprend une durée entre l'événement auriculaire et un événement auriculaire immédiatement précédent ;

**caractérisé en ce que** l'aLP (102a) est en outre configuré pour :

déterminer si une variation associée à l'intervalle AA se situe ou non dans une tolérance spécifiée et, sur cette base, déterminer si un message d'événement auriculaire doit ou non être transmis au vLP pour informer le vLP de l'événement auriculaire ;

s'abstenir de transmettre le message d'événement auriculaire au vLP, lorsqu'il est déterminé que le message d'événement auriculaire ne doit pas être transmis au vLP, pour ainsi s'abstenir d'informer le vLP de l'événement auriculaire ; et

transmettre le message d'événement auriculaire au vLP, lorsqu'il est déterminé que le message d'événement auriculaire doit être transmis au vLP, pour ainsi informer le vLP de l'événement auriculaire.

2. Système implantable (100) selon la revendication 1, dans lequel l'aLP (102a) est configuré pour :

déterminer que le message d'événement auriculaire ne doit pas être transmis au vLP (102b) lorsque l'intervalle AA est déterminé comme étant dans la tolérance spécifiée ; et

déterminer que le message d'événement auriculaire doit être transmis au vLP (102b) lorsque l'intervalle AA n'est pas déterminé comme étant dans la tolérance spécifiée.

3. Système implantable (100) selon la revendication 1, dans lequel l'aLP (102a) est configuré pour :

déterminer que le message d'événement auriculaire ne doit pas être transmis au vLP (102b) lorsque l'intervalle AA et un intervalle AA immédiatement précédent sont chacun dans la tolérance spécifiée ; et

déterminer que le message d'événement auriculaire doit être transmis au vLP (102b) lorsqu'au moins l'un de l'intervalle AA ou de l'intervalle AA immédiatement précédent n'est pas dans la tolérance spécifiée.

4. Système implantable (100) selon l'une quelconque des revendications 1 à 3, dans lequel l'aLP (102a) est configuré pour :

déterminer si un message d'événement auriculaire respectif doit ou non être transmis au vLP (102b) pour les événements auriculaires intrinsèques détectés par l'aLP (102a) ; et

déterminer qu'un message d'événement auriculaire respectif doit être transmis au vLP (102b) pour les événements auriculaires stimulés provoqués par l'aLP (102a).

5. Système implantable (100) selon l'une quelconque des revendications 1 à 3, dans lequel l'aLP (102a) est configuré pour déterminer si un message d'événement auriculaire respectif doit ou non être transmis au vLP (102b) pour des événements auriculaires intrinsèques détectés par l'aLP (102a) et pour des événements auriculaires stimulés provoqués par l'aLP (102a).

6. Système implantable (100) selon l'une quelconque des revendications 1 à 5, dans lequel la vLP (102b) est configurée pour :

utiliser un temporisateur d'intervalle ventriculaire à ventriculaire (intervalle VV) (115) pour déterminer si un intervalle cible ventriculaire s'est écoulé ou non depuis qu'un événement ventriculaire le plus récent s'est produit ; et

régler le temporisateur d'intervalle VV (115) sur l'intervalle cible ventriculaire lorsque le temporisateur d'intervalle VV (115) est réinitialisé.

7. Système implantable (100) selon la revendication 6, dans lequel l'aLP (102a) est configuré pour inclure une mise à jour de l'intervalle cible ventriculaire dans le message d'événement auriculaire transmis par l'aLP (102a) au vLP (102b).

8. Système implantable (100) selon l'une quelconque des revendications 6 ou 7, dans lequel le vLP (102b) est configuré pour :

délivrer une stimulation cardiaque à la cavité ventriculaire, en réponse à l'expiration du temporisateur d'intervalle VV (115) sans que le vLP (102b) reçoive un message d'événement auriculaire de l'aLP (102a) pendant l'intervalle ventriculaire cible.

9. Système implantable (100) selon l'une quelconque des revendications 6 à 8, dans lequel le vLP (102b) est configuré pour :

déterminer si des instances du message d'événement auriculaire ont été reçues ou non de l'aLP (102a) au cours des deux cycles cardiaques consécutifs les plus récents ; et
lorsque des instances du message d'événement auriculaire ont été reçues de l'aLP (102a) au cours des deux cycles cardiaques consécutifs les plus récents, mettre à jour l'intervalle cible ventriculaire en fonction d'un intervalle entre le moment où les instances du message d'événement auriculaire ont été reçues de l'aLP (102a) au cours des deux cycles cardiaques consécutifs les plus récents.

10. Système implantable (100) selon l'une quelconque des revendications 6 à 9, dans lequel le vLP (102b) est configuré pour :

utiliser un temporisateur d'intervalle auriculo-ventriculaire (intervalle AV) (115) pour déterminer quand un intervalle AV spécifié s'est écoulé depuis qu'un événement auriculaire le plus récent s'est produit ;
démarrer le temporisateur d'intervalle AV et annuler le temporisateur d'intervalle VV (115) en réponse à la réception d'un message d'événement auriculaire provenant de l'aLP (102a) ; et
délivrer une stimulation cardiaque à la cavité ventriculaire en réponse à l'expiration du temporisateur d'intervalle AV (115).

11. Système implantable (100) selon l'une quelconque des revendications 1 à 10, dans lequel l'aLP (102a) est configuré pour déterminer si la variation associée à un intervalle AA est ou non dans la tolérance spécifiée par :

déterminer un delta courant indiquant une différence cumulative entre chaque paire consécutive d'intervalles AA déterminés depuis que l'aLP (102a) a transmis le plus récemment le message d'événement auriculaire au vLP (102b) ; et
déterminer que la variation associée à l'intervalle AA est dans la tolérance spécifiée lorsque le delta courant est dans la tolérance spécifiée.

12. Système implantable (100) selon l'une quelconque des revendications 1 à 10, dans lequel l'aLP (102a) est configuré pour déterminer si la variation associée à un intervalle AA est ou non dans la tolérance spécifiée par :

la détermination d'un intervalle cumulatif entre l'intervalle AA et un intervalle AA correspondant au moment où l'aLP (102a) a transmis le plus récemment le message d'événement auriculaire au vLP (102b) ;
la détermination, en fonction de l'intervalle cumulatif et de l'intervalle AA, d'une valeur d'écart indiquant dans quelle mesure les intervalles AA survenant pendant l'intervalle cumulatif s'écartent de l'intervalle AA correspondant au moment où l'aLP (102a) a transmis le plus récemment le message d'événement auriculaire au vLP (102b) ; et
la détermination que la variation associée à l'intervalle AA est dans la tolérance spécifiée lorsque la valeur d'écart est dans la tolérance spécifiée.

13. Système implantable (100) selon l'une quelconque des revendications 1 à 10, dans lequel l'aLP (102a) est configuré pour déterminer si la variation associée à un intervalle AA est ou non dans la tolérance spécifiée par :

la détermination d'un delta entre l'intervalle AA et un intervalle AA correspondant à un événement auriculaire immédiatement précédent ; et
la détermination que la variation associée à l'intervalle AA est dans la tolérance spécifiée lorsque le delta est dans la tolérance spécifiée.

14. Système implantable (100) selon l'une quelconque des revendications 1 à 13, dans lequel l'aLP (102a) s'abstenant de transmettre le message d'événement auriculaire au vLP (102b) réduit une quantité d'énergie consommée par l'aLP (102a) par rapport au moment où l'aLP (102a) transmet le message d'événement auriculaire au vLP (102b).

15. Système implantable (100) selon l'une quelconque des revendications 1 à 14, dans lequel l'aLP (102a) est configuré pour utiliser une communication conductrice, une communication radiofréquence (RF) ou une communication inductive pour transmettre les messages d'événements auriculaires et d'autres types de messages au vLP.

**FIG. 1**

**FIG. 2**

**FIG. 3**

Pace Pulse

404

$T_{pace}$

$T_{i2iP}$

$T_{delayp}$

Tx i2i Comm

406
402

Tx i2i Comm (detail)

408
410

$T_{i2iLF}$

$T_{i2iGap}$

$T_{i2iHF}$

**FIG. 4**

500

Sense Event

Sense threshold

504

502

$T_{delayS}$

$T_{i2iS}$

Tx i2i Comm

506

**FIG. 5**

aLP

vLP

$A_1$  $A_2$

$A_{3a}$  $A_{3b}$  $A_{3c}$

$V_1$  $V_2$  $V_3$

$A_1 V_1$

$V_1 A_2$

$A_2 V_2$

$V_2 A_{3a}$  $V_2 A_{3b}$  $V_2 A_{3c}$

$\Delta AV$

$A_1 A_2$ Interval

$V_1 V_2$ Interval

$V_2 V_3$ Interval

$A_2 A_{3a}$ Interval ($= A_1 A_2 - \Delta AV$)
$A_2 A_{3b}$ Interval ($= A_1 A_2$)
$A_2 A_{3c}$ Interval ($= A_1 A_2 + \Delta AV$)

$A_{3a} V_3$ Interval ($= A_2 V_2 + \Delta AV$)
$A_{3b} V_3$ Interval ($= A_2 V_2$)
$A_{3c} V_3$ Interval ($= A_2 V_2 - \Delta AV$)

*FIG. 6*

Performed by aLP

Initialize AA interval timer — 702

Start AA interval timer
(in response to intrinsic or paced atrial event) — 704

Monitor for intrinsic atrial event — 706

Detect intrinsic atrial event? — 708 — Yes

No

Has AA interval timer expired? — 710 — No

Yes

Determine AA interval — 712

Determine variation associated with AA interval — 714

Variation associated with AA interval within specified tolerance? — 716 — Yes

No

Transmit atrial event message to vLP — 718

Was intrinsic atrial event detected? — 720 — Yes

No

Pace atrial chamber — 722

Reset and Restart AA interval timer — 724

*FIG. 7A*

Performed by aLP

**702**
Initialize AA interval timer

**704**
Start AA interval timer
(in response to intrinsic or paced atrial event)

**706**
Monitor for intrinsic atrial event

**708**
Detect intrinsic atrial event?　Yes

No

**710**
No　Has AA interval timer expired?

Yes

**712**
Determine AA interval

**714**
Determine variation associated with AA interval

**716**
Variation associated with AA interval within specified tolerance?　Yes

**717**
Variation associated with immediately preceding AA interval also within specified tolerance?　Yes

No

No

**718**
Transmit atrial event message to vLP

**720**
Was intrinsic atrial event detected?　Yes

No

**722**
Pace atrial chamber

**724**
Reset and Restart AA interval timer

*FIG. 7B*

Performed by aLP 702

Initialize AA interval timer

704

Start AA interval timer
(in response to intrinsic or paced atrial event)

Monitor for intrinsic atrial event 706

Detect intrinsic atrial event? 708 — Yes

No 710

Has AA interval timer expired? — No

Yes

Transmit atrial event message to vLP 718

Pace atrial chamber 722

Determine AA interval 712

Determine variation associated with AA interval 714

Was intrinsic atrial event detected? 720 — No

Yes

Variation associated with AA interval within specified tolerance? 716 — Yes

No

Transmit atrial event message to vLP 718

Reset and Restart AA interval timer 724

FIG. 7C

Performed by aLP 702

Initialize AA interval timer

704

Start AA interval timer
(in response to intrinsic or paced atrial event)

Monitor for intrinsic atrial event 706

Detect intrinsic atrial event? 708 → Yes

No

Has AA interval timer expired? 710 → No

Yes

Transmit atrial event message to vLP 718

722

Pace atrial chamber

712

Determine AA interval

Determine variation associated with AA interval 714

Was intrinsic atrial event detected? 720 → No

Yes

Variation associated with AA interval within specified tolerance? 716 → Yes → Variation associated with immediately preceding AA interval also within specified tolerance? 717 → Yes

No

No

Transmit atrial event message to vLP 718

724

Reset and Restart AA interval timer

*FIG. 7D*

Embodiment for aLP determining (at step 716) whether variation associated with AA interval is within specified tolerance

New $\Delta$ = AA interval − Immediately Preceding AA interval — 802

Running $\Delta$ = Running $\Delta$ + New $\Delta$ — 804

-$\Delta$ thresh. < Running $\Delta$ < +$\Delta$ thresh. ? — 806

Yes

No

Conclude that Variation associated with AA interval is within specified tolerance (and do not reset Running Delta) — 808

Conclude that Variation associated with AA interval is not within specified tolerance (and reset Running Delta = 0) — 810

*FIG. 8*

Embodiment for aLP determining (at step 716)
whether variation associated with AA interval
is within specified tolerance

Determine Cumulative Interval between AA interval and AA interval corresponding to when atrial event message most recently transmitted by aLP to vLP _902

Determine, based on Cumulative Interval and AA interval, a Deviation Value (DV) indicative of how much AA intervals occurring during Cumulative Interval deviate from AA interval corresponding to when aLP most recently transmitted atrial event message to vLP _904

906

Yes  -Δ thresh. < DV < +Δ thresh. ?  No

908

Conclude that Variation associated with AA interval is within specified tolerance

910

Conclude that Variation associated with AA interval is not within specified tolerance

FIG. 9

Performed by vLP — 1002

Initialize Ventricular Target Interval
(e.g., to equal Base Rate or Sensor Indicated Rate (SIR))

— 1004

Start (or Restart) VV interval timer
with starting value of Ventricular Target Interval
(and Cancel AV interval timer if it had been started)

Monitor for intrinsic ventricular event — 1006

Monitor for atrial event message from aLP — 1016

Detect intrinsic ventricular event? — 1008
Yes / No

Atrial event message received from aLP? — 1018
Yes / No

VV interval timer canceled? — 1010
Yes / No

Start AV interval timer and Cancel VV interval timer — 1020

VV interval timer expired? — 1012
No / Yes

AV interval timer expired? — 1022
No / Yes

Pace ventricular chamber — 1024

Atrial event messages received from aLP during two most recent consecutive cardiac cycles? — 1026
No / Yes

Determine interval between instances of Atrial event messages received from aLP during two most recent consecutive cardiac cycles — 1028

Redefine Ventricular Target Interval to be equal to interval between instances of Atrial event messages received from aLP during two most recent consecutive cardiac cycles — 1030

*FIG. 10*

**FIG. 11**

EP 4 487 902 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2021205628 A1
- US 10182765 B
- US 10993674 B